(19) Europäisches Patentamt
European Patent Office
Office européen des brevets

(11) EP 0 362 860 B1

(12) EUROPEAN PATENT SPECIFICATION

(45) Date of publication and mention of the grant of the patent:
07.02.1996 Bulletin 1996/06

(51) Int Cl.6: **C08G 77/38**, C08G 77/46, A61K 7/00

(21) Application number: 89118531.6

(22) Date of filing: 05.10.1989

# (54) Alcohol-modified silicone ester derivative and cosmetic composition containing same

Esterderivate von mit Alkohol modifizierten Siloxanen und kosmetische Zusammensetzungen, die diese Derivate enthalten

Ester dérivé d'un siloxane ayant des groupes alcools, compositions cosmétiques contenant ces dérivés

(84) Designated Contracting States:
**DE ES FR**

(30) Priority: **07.10.1988 JP 252942/88**
**05.12.1988 JP 307456/88**
**27.02.1989 JP 45795/89**

(43) Date of publication of application:
**11.04.1990 Bulletin 1990/15**

(73) Proprietor: **KAO CORPORATION**
**Chuo-ku, Tokyo (JP)**

(72) Inventors:
- **Kawamata, Akira**
**Utsunomiya-shi, Tochigi (JP)**
- **Kikuchi, Yoko**
**Toride-shi, Ibaraki (JP)**
- **Suzuki, Yuji**
**Sakura-shi, Chiba (JP)**
- **Suzuki, Toshiyuki**
**Ichikawa-shi, Chiba (JP)**
- **Suda, Mituo**
**Ichikaimachi, Haga-gun, Tochigi (JP)**
- **Ohashi, Yukihiro**
**Haga-gun, Tochigi (JP)**

(74) Representative:
**Wächtershäuser, Günter, Prof. Dr.**
**D-80331 München (DE)**

(56) References cited:

| | |
|---|---|
| EP-A- 0 186 507 | EP-A- 0 252 878 |
| EP-A- 0 297 614 | DE-A- 1 467 859 |
| DE-A- 1 916 015 | US-A- 4 587 320 |

- **PATENT ABSTRACTS OF JAPAN, vol. 12, no. 410 (C-540)[3257], 28th October 1988; & JP-A-63 150 288 (NISSHIN OIL MILLS LTD) 22-06-1988**

Remarks:
The file contains technical information submitted after the application was filed and not included in this specification

EP 0 362 860 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

BACKGROUND OF THE INVENTION

i) Field of the Invention:

This invention relates to a novel alcohol-modified silicone ester derivative (hereinafter referred to simply as silicone ester derivative) and cosmetic compositions containing same.

ii) Description of the Background Art:

Silicone oils are widely used as an oil ingredient for cosmetic compositions due to their excellent lubricity, water repellency, gloss imparting capability, stability, safety, etc. Among them, a high molecular dimethylpolysiloxane has generally been used in the art. The dimethylpolysiloxane, however, raises a problem when incorporated in a cosmetic composition. It gives disagreeable feel to the skin. In detail, it gives creaky feel when that of low viscosity is used and sticky feel when that of high viscosity is used. Moreover, it is accompanied by an another problem in that an emulsion system is difficult to obtain because the high molecular dimethylpolysiloxane has poor compatibility with hydrocarbon oils.

With regard to the art focusing on the above problem of the high molecular dimethylpolysiloxane, there are two publications which are Japanese patent applications laid-open to the public, Kokai 61-129187 and Kokai 63-150288. They disclose esterified products between dimethylpolysiloxanediol and a dibasic acid or between dimethyl polysiloxanediol and a long-chain fatty acid, respectively. These compounds, however, still demand improvement of feel on use and blenbability.

The present invention was made primarily to overcome the above-mentioned drawbacks of the silicone oils by taking an approach quite different from those disclosed in the mentioned publications, and to develop a novel silicone derivative having excellent properties which were never obtained from the conventional silicone oils, and further to develop cosmetic compositions having agreeable feel on use.

The present inventors carried out an earnest study focusing on the above goals and found that an alcohol-modified silicone ether derivative of formula (I) had a good compatibility with other solvents, gave agreeable feel upon use and was free from the drawbacks accompanied by the conventional silicone oils. Such findings have led to the completion of this invention.

SUMMARY OF THE INVENTION

Accordingly, an object of this invention is to provide an alcohol-modified silicone ester derivative represented by the following formula (I):

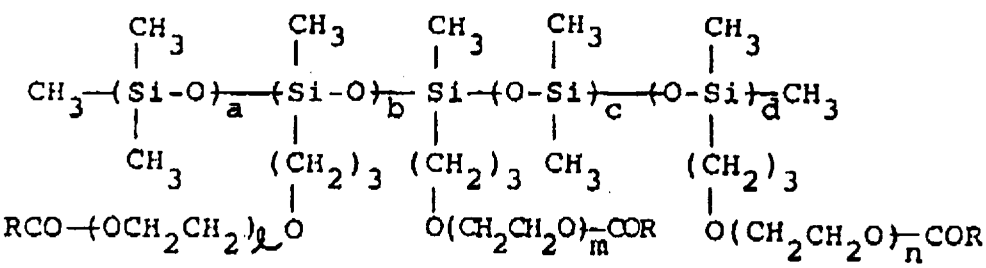

(I)

wherein RCO is selected from abietic acid residue, dehydroabietic acid residue, dihydroabietic acid residue, tetrahydroabietic acid residue and cholic acid residue; a, b and c are numbers which sum falls in the range from 0 to 130; d is 0 and 1 provided that a= b= c= d= 0 is excluded; and 1, m and n are numbers which sum falls in the range from 0 to 6.

Another object of this invention is to provide a cosmetic composition containing the same.

Brief Description of the Drawings

Fig. 1 shows an infrared absorption spectrum of the silicone tetrahydroabietic ester derivative obtained in Example 1.

Fig. 2 shows a $^{1}$H-NMR spectrum of the silicone tetrahydroabietic ester derivative obtained in Example 1.

Fig. 3 shows a $^{13}$C-NMR spectrum of the silicone tetrahydroabietic ester derivative obtained in Example 1.

Fig. 4 shows a $^{29}$Si-NMR spectrum of the silicone tetrahydroabietic ester derivative obtained in Example 1.

Fig. 5 shows an infrared absorption spectrum of the silicone tetrahydroabietic ester derivative obtained in Example 2.

Fig. 6 shows a $^{1}$H-NMR spectrum of the silicone tetrahydroabietic ester derivative obtained in Example 2.
Fig. 7 shows a $^{13}$C-NMR spectrum of the silicone tetrahydroabietic ester derivative obtained in Example 2.
Fig. 8 shows a $^{29}$Si-NMR spectrum of the silicone tetrahydroabietic ester derivative obtained in Example 2.
Fig. 9 shows an infrared absorption spectrum of the silicone tetrahydroabietic ester derivative obtained in Example 9.
Fig. 10 shows a $^{1}$H-NMR spectrum of the silicone tetrahydroabietic ester derivative obtained in Example 9.
Fig. 11 shows a $^{13}$C-NMR spectrum of the silicone tetrahydroabietic ester derivative obtained in Example 9.

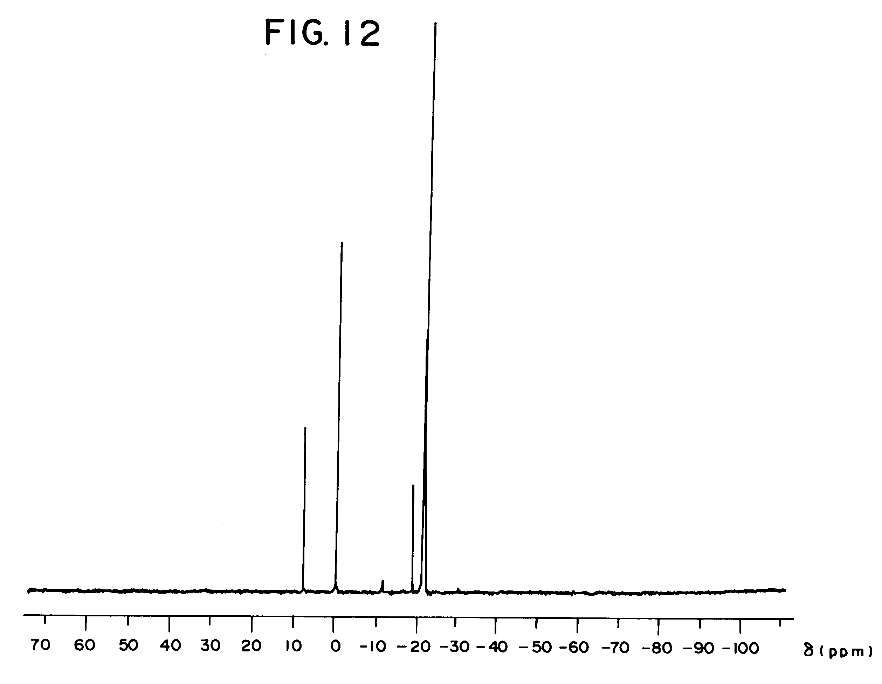

Fig. 12 shows a $^{29}$Si-NMR spectrum of the silicone tetrahydroabietic ester derivative obtained in Example 9.

## DETAILED DESCRIPTION OF THE

## INVENTION AND PREFERRED EMBODIMENTS

The silicone ester derivative of formula (I) can be prepared, for example, by whichever process A or B.

### Process A:

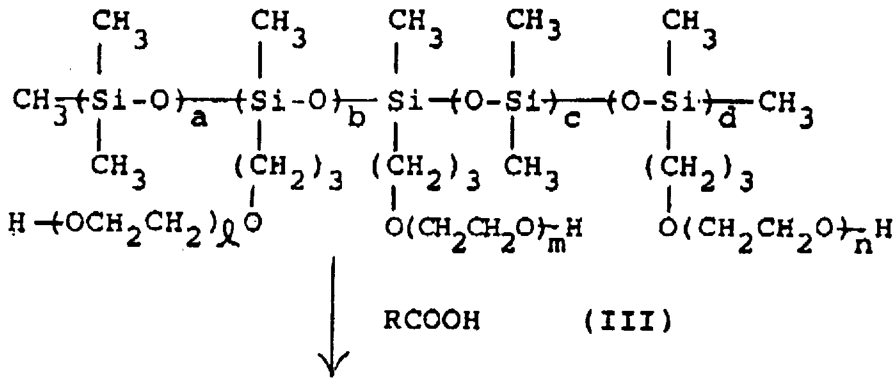


(II)

(I)

Here, R, a, b, c, l, m and n have the same meanings as defined before.

In detail, the silicone ester derivative (I) of the present invention can be prepared by the reaction of alcohol-modified silicone (II) with carboxylic acid (III) or its reactive derivative.

The alcohol-modified silicones (i.e, alcohol-modified dimethylpolysiloxane), the starting material of the above process, are commercially available and sold, for example, by Shin'etsu Kagaku Kogyo K.K. They may however be readily synthesized by the reaction of a corresponding polysiloxane having Si-H terminals with an ethylene oxide adduct of allyl alcohol. The molecular weight of alcohol- or allyl alcohol- modified silicones (II) is preferably 130 to 10,000. Moreover, it is preferred that a and b in formula (II) be zero (0) and d be one (1) in view of the availability, They can be used singly or in combination.

The carboxylic acids(III) are selected from abietic acid, dehydroabietic acid, dyhydroabietic acid, tetrahydroabiectic acid and cholic acid. These carboxylic acids (III) may be used singly or in combination. An example of such combination is a mixed hydrogenated abietic acid which is a mixture of dihydroabietic acid and tetrahydroabietic acid.

The mixed hydrogenated abietic acid is a major component of the rosin, and sold, for example, by Harima Kasei Kogyo K.K. This may otherwise be obtained by a catalytic reduction of abetic acid by the use of a metal catalyst such as palladium, platinum and nickel. Moreover, it is readily obtainable from market as a hydrogenated rosin.

Examples of the reactive derivatives of the carboxylic acids (III) include carboxylic acid halides such as carboxylic acid bromide and carboxylic acid chloride.

The esterification between alcohol-modified silicone (II) and carboxylic acid (III) is carried out according to a known method. For instance, (II) and (III) are mixed for dehydrative condensation under moderate heating in the presence of an acidic catalyst such as tin chloride and toluenesulfonic acid, or at a high temperature in the absence of a catalyst. The reaction between alcohol-modified silicone (II) and carboxylic halide is carried out according to a known method, and for instance, it proceeds in the presence of a basic catalyst such as pyridine at room temperature or under heat.

### Process B:

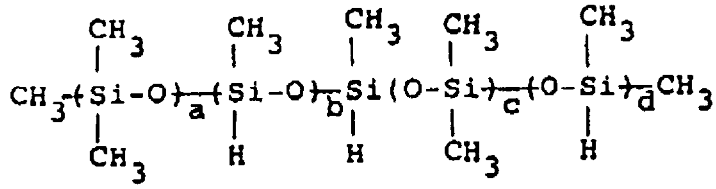

(IV)

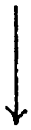

$RCO(OCH_2CH_2)_nOCH_2CH=CH_2$ (V)

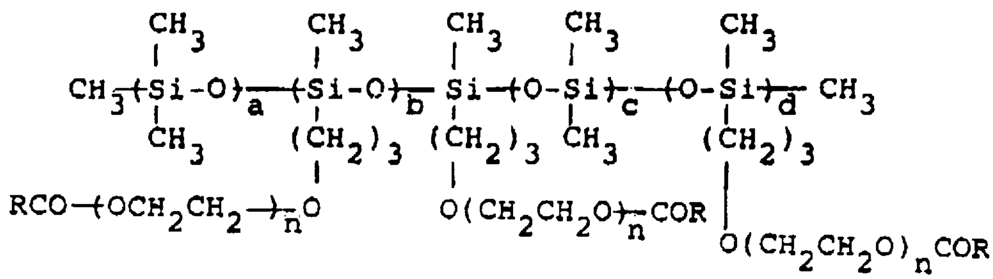

(VI)

Here, R, a, b, c, d and n have the same meanings as mentioned before.

In detail, polysiloxane (IV) having Si-H bonds and carboxylic acid ester (V) are reacted for hydrosilylation at room temperature or under heat in the presence of a catalytic amount of chloroplatinic acid catalyst. The reaction can be carried out either without solvent or using solvent.

The thus obtained silicone ester derivative (I) of the present invention has features: 1) liquid at room temperature, 2) chemically stable, and 3) very mild to the skin; and is very useful as an oil ingredient for any kind of cosmetic compositions, especially for those which are directly applied to the skin.

The amount of the silicone ester derivative (I) to be incorporated in a cosmetic composition is not particularly limited and generally from 0.001 to 90 wt% of the total composition, with a preferable amount being 0.1 to 80 wt% and most preferably 1 to 50 wt%.

When a polyol-type moisturizer is used in combination with the silicone ester derivative (I), moisturizing effect as well as thick and moistened feel are obtained with a reduced stickiness compared to when the respective ingredients are used singly. Examples of the polyol-type moisturizer include propylene glycol, 1,3-butanediol, dipropylene glycol, glycerol, diglycerol, polyglycerol, tritirol propane, erythritol, pentaerythritol, sorbitane, glucose, sorbitol, martitol, saccharose, trehalose, polyoxyethylene methyl glucoside, polyoxypropylene methyl glucoside and polyethylene glycol. Among them, especially preferred are 1,3-butanediol, glycerol, sorbitol and polyoxyethylene methyl glucoside. These polyol-type moisturizers are preferably incorporated in a cosmetic composition from 2 to 90 wt% and more preferably from 5 to 50 wt% based on the total amount. If the amount is less than 2 wt%, advantage of the combination use is not notable, while at an amount exceeding 90 wt%, the moisture-retaining effect is saturated and no further effect will be observed. The blending proportion between the silicone ester derivative (I) and the polyol-type moisturizer is arbitrary.

Aside from the above-indicated ingredients, the cosmetic compositions according to the present invention may further contain, so far as the effects of the invention are not impeded, oils, surfactants, moisturizers, UV absorbers, chelating agents, pH adjusters, preservatives, viscosity colorants, and perfumes which are generally used in the art.

There is no specific restrictions as to the category of the cosmetic compositions or their forms. Examples of the cosmetic compositions include skin-care compositions such as W/O- and O/W- emulsified cosmetic products, creams, lotions and milky lotions, oil-base cosmetic compositions, lipsticks, foundations, skin detergents, hair tonics, hair setting agents, hair growers, hair nourishing agents and so on.

The silicone ester derivative (I) of the present invention is applicable to a variety of blending systems in the art. This may be explained by the fact that there are three different parts in a molecule of the ester derivative (I), which are dimethylpolysiloxisane part, carboxylic hydrophobic part and ester or ethylene dioxy group part, and these three parts are capable of undergoing interaction with silicone oil, hydrocarbon oil and polar solvent or water, respectively.

Thus, when the silicone ester derivative (I) of the present invention is incorporated into any compositions such as

cosmetic or skin-care compositions which are comprised of a hydrocarbon oil, a polar solvent, water and so on, it is capable of maintaining the compositions stable for a prolonged period. Moreover, due to the presence of the ester derivative (I), the cosmetic compositions according to this invention remarkably reduce creaky feel on use which the conventional silicone oils couldn't avoid. Especially, it is notable that when silicone ester derivative (I) and a polyol-type moisturizer are used in combination for preparing a cosmetic composition, excellent properties are obtainable which include enhanced moisture-retainability and thick and moistened feel with supressed stickiness.

EXAMPLES

This invention will now be explained by way of Examples, which should not be construed as limiting the invention thereto.

<u>Example 1.</u>

Tetrahydroabietic Ester Derivative of Alcohol-modified Silicone:

In a 200 ml two-necked flask equipped with a stirrer, dropping funnel and a reflux condenser was placed a 30g(5.35 mmol) of alcohol-modified silicone (product of Shin'etsu Kagaku Kogyo K.K.; a+b=0, c(mean value)=72, d=1, m+n=3 in formula (II)), 0.93g(11.7 mmol) of pyridine and 60g of toluene. The flask was stirred under nitrogen atmosphere at room temperature. 10 ml of toluene containing 3.80g(11.7 mmol) of tetrahydroabietoyl chloride was dropped into the flask over 20 minutes. After the completion of the dropping, the flask was heated at 80°C and subjected to 15 hours consecutive stirring under heat. Thereafter, the flask was allowed to stand for cooling down and 3 ml methanol was added to decompose the remaining tetrahydroabietoyl chloride. The toluene layer was washed four times with 50 ml water. After the solvent was evaporated, the obtained yellow viscous product was subjected to silica gel column chromatography (silica gel: 230-400 mesh, solvents: dichloromethane/hexane = 1 - 4/1, dichloromethane only) to obtain 25.6g of the title compound as a pale-yellow viscous material

( R = 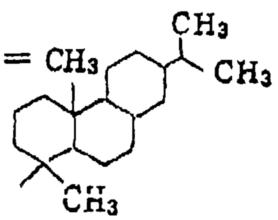 ,

a+b=0, $\bar{c}$ (mean value)=72, d=1, m+n=3 in formula (I)).
Yield:77.5%

IR(liquid film, $cm^{-1}$): 2964, 1732, 1446, 1416, 1262, 1096, 1024, 868, 802, 662 (Fig. 1)

$^1$H-NMR($C_6D_6$, δ) 0.1~0.3($C\underline{H}_3$-Si about 450H) 0.5~2.0 (H derived from tetrahydroabietic acid, Si-$C\underline{H}_2$-$C\underline{H}_2$-$C\underline{H}_2$-O, 76H) 3.2~3.5(Si-$CH_2CH_2$-$C\underline{H}_2$-O-$C\underline{H}_2$-$C\underline{H}_2$-O-$C\underline{H}_2$-,12H) 4.0~4.3($CO_2$-$C\underline{H}_2$-,4H) (Fig. 2)

$^{13}$C-NMR($C_6D_6$, δ) 0.3~1.9($\underline{C}H_3$-Si, $\underline{C}H_2$-Si) 14.6~56.2 ($\underline{C}H_3$ derived from tetrahydroabietic acid, $\underline{C}H_2$, $\underline{C}H$, si$CH_2$-$\underline{C}H_2$-$CH_2$-O) 61.9, 63.7 (Si$CH_2CH_2\underline{C}H_2$-O) 69.0 (-$CH_2O\underline{C}H_2\underline{C}H_2OCH_2$-) 72.2, 74.0 (-$\underline{C}H_2OCO$-) 178.0 (-$\underline{C}OO$-) (Fig. 3)

$$^{29}Si\text{-NMR}(C_6D_6,\ \delta)\ -22.3\sim-20.8(-O-\underset{CH_3}{\overset{CH_3}{\underline{Si}}}-O-)\ 8.13(-O-\underset{CH_3}{\overset{CH_3}{\underline{Si}}}-CH_2-)$$

(Fig. 4)

Example 2

Tetrahydroabietic Ester Derivative of Alcohol-modified Silicone:

The process of Example 1 was followed using 30g (30 mmol) of alcohol-modified silicone (product of Shin'etsu Kagaku Kogyo K.K.; a+b=0, $\bar{c}$ (mean value)=10, d=1, m=n=1 in formula (II)), 10.44g (132 mmol) of pyridine, 60g of toluene and 23.39g (72 mmol) of tetrahydroabietoyl chloride to obtain 33.1g of the title compound as a pale-yellow, viscous material

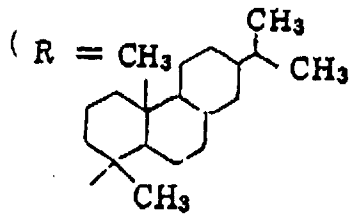

a+b=0, $\bar{c}$ (mean value)=10, d=1, m=n=1 in formula (I)).
Yield: 69.9%

IR(liquid film, $cm^{-1}$) 2962, 2872, 1731, 1455, 1416, 1389, 1371, 1260, 1170, 1095, 1038, 801, 702, 687, 666 (Fig. 5) $^1$H-NMR($C_6D_6$, δ)0.1~0.3($\underline{CH_3}$-Si about 72H) 0.5~2.0(H derived from tetrahydroabietic acid, Si-C$\underline{H_2}$-C$\underline{H_2}$-C$H_2$O-C, 76H) 3.24, 3.37(SiCH$_2$CH$_2$-C$\underline{H_2}$-O-C$\underline{H_2}$- 4H each) 4.0~4.2(-C$\underline{H_2}$OCO-, 4H) (Fig. 6) $^{13}$C-NMR($C_6D_6$, δ) 0.3~1.5($\underline{C}$H$_3$-Si, $\underline{C}$H$_2$-Si) 14.6~56.9 ($\underline{C}$H$_3$ derived from tetrahydroabietic acid, $\underline{C}$H$_2$, $\underline{C}$H, Si-CH$_2$-$\underline{C}$H$_2$-CH$_2$-O) 63.6(SiCH$_2$CH$_2$-$\underline{C}$H$_2$-O) 69.0(SiCH$_2$CH$_2$CH$_2$O-$\underline{C}$H$_2$-CH$_2$) 73.9(-$\underline{C}$H$_2$OCO-) 178.0(-$\underline{C}$OO-) (Fig. 7)

CH3 CH3
$^{29}$Si-NMR($C_6D_6$, δ) -22.3~-20.7(-O-$\underline{Si}$-O-) 8.14 (-O-$\underline{Si}$-CH$_2$-)
CH3 CH3

(Fig. 8)

Examples 3-8

The process of Example 1 was followed to obtain silicone ester derivatives (I) tabulated in Table 1.

TABLE 1

| Example Nos. | RCO- | a+b | c (mean value) | d | l | m+n |
|---|---|---|---|---|---|---|
| Ex. 3 | Tetrahydroabietic acid residue | 0 | 50 | 1 | 0 | 3 |
| Ex. 4 | Dehydroabietic acid residue | 0 | 12 | 1 | 0 | 2 |
| Ex. 5 | Dehydroabietic acid residue | 0 | 21 | 1 | 0 | 2 |
| Ex. 6 | Cholic acid residue | 0 | 50 | 1 | 0 | 2 |
| Ex. 7 | Abietic acid residue | 0 | 70 | 1 | 0 | 4 |
| Ex. 8 | Dihydroabietic acid residue | 0 | 25 | 1 | 0 | 2 |

Example 9

Synthesis of Tetrahydroabietic Ester Derivative of Alcohol-modified Silicone (RCO=tetrahydroabietic acid residue, a=b=0, $\bar{c}$ (mean value)=14, d=1 in formula (VI)):

In a 30 ml round bottomed flask, 14.04g (12.0 mmol) of siloxane oligomer (product of Toshiba Silicone K.K.; a=b=0,

c=14, d=1), 7.00g (20.2 mmol) of allyl tetrahydroabietate and a catalyst amount of chloroplatinic acid·$6H_2O$ were placed and mixed. After stirring over 24 hours at room temperature, 100 ml of hexane was added, and the hexane layer was washed three times with 50 ml water. The hexane layer was dried over sodium sulfate and the solvent was evaporated. A dark brown oily material was obtained. This material was purified through medium-pressure liquid chromatography (Silica gel 25~40μm 350g; hexane/ethyl acetate = 29/1) to obtain 14.74g of the title compound as a pale-yellow oily material. Yield: 77.7%

IR(liquid film, $cm^{-1}$) 2964, 2872, 1730, 1580, 1540, 1450, 1414, 1388, 1262, 1104, 1032, 864, 810, 702, 664 (Fig. 9)

$^1H$-NMR($C_6D_6$, δ) 0.1-0.3(C<u>H</u>$_3$-Si, about 96H) 0.5-2.0(H derived from tetrahydroabietic acid, Si-C<u>H</u>$_2$-C<u>H</u>$_2$-$CH_2$-O, 74H), 4.01(-C<u>H</u>$_2$-$O_2$C-, 4H) (Fig. 10)

$^{13}C$-NMR($C_6D_6$, δ) 0.3-1.5($CH_3$-Si, $CH_2$-Si), 14.5-57.0(<u>C</u>H$_3$ derived from tetrahydroabietic acid, <u>C</u>H$_2$, <u>C</u>H, Si-$CH_2$-<u>C</u>H$_2$-$CH_2$), 66.9(-<u>C</u>H$_2$-$O_2$C- ), 178.0(-<u>C</u>OO-) (Fig. 11)

$^{29}Si$-NMR($C_6D_6$, δ) -22.1--18.7(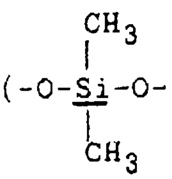) 7.90(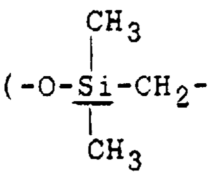)

(Fig. 12)

<u>Examples 10-22</u>

The process of Example 9 was followed to obtain silicone ester derivatives (I) tabulated in Table 2.

TABLE 2

| Example Nos. | RCO- | a | b | c | d | l | m | n |
|---|---|---|---|---|---|---|---|---|
| Ex. 10 | Tetrahydroabietic acid residue | a + c = 40 b = 4, d = 0 | | | | 0 | 0 | 0 |
| Ex. 11 | Dihydroabietic acid residue | a = b = 0, c = 22, d = 1 | | | | 0 | 0 | 0 |
| Ex. 12 | Dihydroabietic acid residue | a + c = 14, b = 2, d = 0 | | | | 0 | 0 | 0 |
| Ex. 13 | Dihydroabietic acid residue | a + c = 80, b = 20, d = 0 | | | | 0 | 0 | 0 |
| Ex. 14 | Tetrahydroabietic acid residue | a + c = 42, b = 10, d = 0 | | | | 0 | 0 | 0 |
| Ex. 15 | Tetrahydroabietic acid residue | a + c = 14, b = 3, d = 0 | | | | 0 | 0 | 0 |
| Ex. 16 | Dihydroabietic acid residue | a + c = 22, b = 10, d = 0 | | | | 0 | 0 | 0 |
| Ex. 17 | Tetrahydroabietic acid residue | a = b = 0, c = 50, d = 1 | | | | 0 | 0 | 0 |
| Ex. 18 | Tetrahydroabietic acid residue | a = b = 0, c = 72, d = 1 | | | | 0 | 0 | 0 |
| Ex. 19 | Tetrahydroabietic acid residue | a + c = 60, b = 5, d = 0 | | | | 0 | 0 | 0 |
| Ex. 20 | Tetrahydroabietic acid residue | a + c = 12, b = 4, d = 0 | | | | 0 | 0 | 0 |
| Ex. 21 | Tetrahydroabietic acid residue | a + c = 12, b = 8, d = 0 | | | | 0 | 0 | 0 |
| Ex. 22 | Dihydroabietic acid residue | a + c = 36, b = 8, d = 0 | | | | 0 | 0 | 0 |

* All values are mean values.

Example 23

Emulsified Cosmetic Composition:

Emulsified cosmetic compositions shown in Table 3 were prepared according to the process below. Evaluation was made by ten(10) expert panelists, which results are also shown in Table 3.

Process

Ingredients 1) to 4) in Table 3 were mixed under heat, to which a mixture of 5) and 6) were added and stirred to obtain a substantially homogeneous solution. Comparative products were prepared by the same manner.

TABLE 3

| | Emulsified cosmetic composition | Invention Product 1 | Invention Product 2 | Comparative Product 1 | Product 1a* | Product 2a* |
|---|---|---|---|---|---|---|
| Composition (%) | 1) Silicone ester derivative (I) (Example 3; R=tetrahydroabietic acid residue, a+b=0, c=50, d=1, m+n=3, l=0) | 10.0 | -- | -- | 10.0 | -- |
| | 2) Silicone ester derivative (I) (Example 1; R=tetrahydroabietic acid residue, a+b=0, c=72, d=1, m+n=3, l=0) | -- | 10.0 | -- | -- | 10.0 |
| | 3) Squalane | -- | -- | 10.0 | -- | -- |
| | 4) Polyoxyethylene oleylether (20E.O.) | 2.0 | 2.0 | 2.0 | 2.0 | 2.0 |
| | 5) Glycerol | 8.0 | 8.0 | 8.0 | -- | -- |
| | 6) Purified water | Balance | Balance | Balance | Balance | Balance |
| Feel on Use | Moistness | Yes (moistened) | Yes | Yes | No | No |
| | Stickiness | slightly unsticky | slightly unsticky | Yes (sticky) | No | No |
| | Smoothness | Yes (smooth) | Yes | No | Yes | Yes |
| | Effectiveness for chapped skin | Yes (effective) | Yes | Yes | slightly uneffective | slightly uneffective |

*without glycerol moisturizer

Examples 24 Moisturizing Cream:

Moisturizing creams formulated as shown in Table 4 were prepared according to the process below. Evaluation was made by ten(10) expert panelists, which results are also shown in Table 4.

Process

Ingredients 1) to 8) and 11) to 13) in Table 4 were allowed to dissolve under heat, and the temperature was maintained at 70°C. Ingredients 9), 10) and 15) were also mixed at 70°C, to which the mixture of 1) to 8) and 11) to 13) was added. The obtained mixture was charged in an emulsifier for emulsification. The resultant emulsion was added with ingredient 14) and cooled down to 30°C by a heat exchanger to obtain a moisturizing cream.

TABLE 4

| | Moisturizing cream | Invention Product 3 | Invention Product 4 | Comparative Product 4 |
|---|---|---|---|---|
| Composition (%) | 1) Silicone ester derivative (I) (Ex. 4, R=dehydroabietic acid residue, a+b=0, c=12, d=1, m+n=2, l=0) | 8.0 | -- | -- |
| | 2) Silicone ester derivative (I) (Ex. 5, R=dehydroabietic acid residue, a+b=0, c=21, d=1, m+n=2, l=0) | -- | 8.0 | -- |
| | 3) Liquid paraffin | -- | -- | 8.0 |
| | 4) Beeswax | 2.0 | 2.0 | 2.0 |
| | 5) Stearyl alcohol | 3.0 | 3.0 | 3.0 |
| | 6) Stearic acid | 5.0 | 5.0 | 5.0 |
| | 7) Squalane | 7.0 | 7.0 | 7.0 |
| | 8) Polyoxyethylene cetylether (20E.O.) | 1.0 | 1.0 | 1.0 |
| | 9) Propylene glycol | 8.0 | 8.0 | 8.0 |
| | 10) Glycerol | 4.0 | 4.0 | 4.0 |
| | 11) Triethanolamine | 1.0 | 1.0 | 1.0 |
| | 12) Methyl paraben | 0.1 | 0.1 | 0.1 |
| | 13) Butyl paraben | 0.1 | 0.1 | 0.1 |
| | 14) Perfume | 0.1 | 0.1 | 0.1 |
| | 15) Purified water | Balance | Balance | Balance |
| Feel on Use | Moistness | Yes (moistened) | Yes | slightly moistened |
| | Stickiness | No | No | Yes (sticky) |
| | Smoothness | Yes (smooth) | Yes | No |
| | Effectiveness for chapped skin | Yes (effective) | Yes | slightly effective |

Example 25

Emulsified Cosmetic Composition:

Emulsified cosmetic compositions shown in Table 5 were prepared according to the process below. Evaluation was made by ten(10) expert panelists, which results are also shown in Table 5.

Process

Ingredients 1) to 4) in Table 5 were mixed under heat and added with a mixture of 5) and 6) to obtain a substantially homogeneous solution by stirring. Comparative products were prepared by the same manner.

TABLE 5

| | Emulsified cosmetic composition | Invention Product 5 | Invention Product 6 | Comparative Product 5 | Product 5a* | Product 6a* |
|---|---|---|---|---|---|---|
| Composition (%) | 1) Silicone ester derivative (I) (Ex.9) | 10.0 | -- | -- | 10.0 | -- |
| | 2) Silicone ester derivative (I) (Ex.10) | -- | 10.0 | -- | -- | 10.0 |
| | 3) Liquid paraffin | -- | -- | 10.0 | -- | -- |
| | 4) Polyoxyethylene oleyl ether (20 E.O.) | 2.0 | 2.0 | 2.0 | 2.0 | 2.0 |
| | 5) Glycerol | 8.0 | 8.0 | 8.0 | -- | -- |
| | 6) Purified water | Balance | Balance | Balance | Balance | Balance |
| Feel on Use | Moistness | Yes (moistened) | Yes | Yes | No | No |
| | Stickiness | slightly unsticky | slightly unsticky | Yes (sticky) | No | No |
| | Smoothness | Yes (smooth) | Yes | No | Yes | Yes |
| | Effectiveness for chapped skin | Yes (effective) | Yes | Yes | slightly uneffective | slightly uneffective |

*without glycerol moisturizer

Example 26 Moisturizing Cream:

Moisturizing creams formulated as shown in Table 6 were prepared according to the process below. Evaluation was made by ten(10) panelists, which results are also shown in Table 6.

Process

Ingredients 1) to 8) and 11) to 13) in Table 6 were allowed to dissolve under heat, and the temperature was maintained at 70°C. In the same manner, ingredients 9), 10) and 15) were heated and mixed, to which the mixture of 1) to 8) and 11) to 13) was added. The obtained mixture was charged in an emulsifier for emulsification. Ingredient 14) was added to the resultant emulsion and cooled down to 30°C by a heat exchanger to obtain a moisturizing cream.

TABLE 6

| | Moisturizing cream | Invention Product 7 | Invention Product 8 | Comparative Product 8 |
|---|---|---|---|---|
| Composition (%) | 1) Silicone ester derivative (I) (Ex.11) | 8.0 | -- | -- |
| | 2) Silicone ester derivative (I) (Ex.12) | -- | 8.0 | -- |
| | 3) Liquid paraffin | -- | -- | 8.0 |
| | 4) Bees wax | 2.0 | 2.0 | 2.0 |
| | 5) Stearyl alcohol | 3.0 | 3.0 | 3.0 |
| | 6) Stearic acid | 5.0 | 5.0 | 5.0 |
| | 7) Squalane | 7.0 | 7.0 | 7.0 |
| | 8) Polyoxyethylene cetyl ether (20 E.O.) | 1.0 | 1.0 | 1.0 |
| | 9) Propylene glycol | 8.0 | 8.0 | 8.0 |
| | 10) Glycerol | 4.0 | 4.0 | 4.0 |
| | 11) Triethanolamine | 1.0 | 1.0 | 1.0 |
| | 12) Methyl paraben | 0.1 | 0.1 | 0.1 |
| | 13) Butyl paraben | 0.1 | 0.1 | 0.1 |
| | 14) Perfume | 0.1 | 0.1 | 0.1 |
| | 15) Purified water | Balance | Balance | Balance |
| Feel on Use | Moistness | Yes (moistened) | Yes | slightly moistened |
| | Stickiness | No | No | Yes (sticky) |
| | Smoothness | Yes (smooth) | Yes | No |
| | Effectiveness for chapped skin | Yes (effective) | Yes | slightly effective |

Example 27 Cold Cream:

The tetrahydroabietic ester derivative (I) obtained in Example 1 was used for preparing a cold cream.

(Composition)

| | |
|---|---|
| 1) Tetrahydroabietic ester derivative (Example 1) | 5.0 wt% |
| 2) Silicone oil | 5.0 |
| 3) Liquid paraffin | 10.0 |
| 4) Bees wax | 3.0 |
| 5) Lanolin | 1.5 |
| 6) Polyoxyethylene sorbitan monooleate | 3.0 |
| 7) Sorbitan monooleate | 2.0 |
| 8) Methyl paraben | 0.1 |
| 9) Buthyl paraben | 0.1 |
| 10) Perfume | 0.2 |
| 11) Purified water | Balance |

(Preparation)

Ingredients 1) to 7) and 9) were mixed at 70°C and then added with a 70'C mixture of 8) and 11) under stirring to obtain an emulsion. The emulsion was allowed to cool down to about 40°C, to which ingredient 10) was added to obtain a cream. This cream has notable extendability and fittability on the skin, and was suitable as a cold cream.

Example 28

Creamy Foundation:

The tetrahydroabietic ester derivative (I) obtained in Example 1 was used for preparing a creamy foundation having the following composition.

(Composition)

| | |
|---|---|
| 1) Tetrahydroabietic ester derivative (Example 1) | 1.5 wt% |
| 2) Polyoxyethylene stearate | 2.5 |
| 3) Isopropyl myristate | 6.0 |
| 4) Stearic acid | 5.0 |
| 5) Talc | 12.0 |
| 6) Titanium oxide | 5.0 |
| 7) Red ion oxide | 0.5 |
| 8) Methyl paraben | 0.1 |
| 9) Propyl paraben | 0.1 |
| 10) Perfume | 0.2 |
| 11) Purified water | Balance |

(Preparation)

Ingredients 1) to 4) and 9) were mixed at 70°C and then added with a 70°C mixture of 8) and 11) under stirring to obtain an emulsion. The emulsion was further blended with ingredients 5) to 7), and stirred and mixed. The mixture was cooled down to 40°C, when ingredient 10) was added thereto. The thus obtained emulsion product was resistant to perspiration and was capable retaining the make-up for a prolonged time, thus revealed excellent properties when used as a make-up foundation.

Example 29

Toilet lotion:

The tetrahydroabietic ester derivative (I) obtained in Example 1 was used or preparing a toilet lotion having the

following composition.

(Composition)

| | |
|---|---|
| 1) Tetrahydroabietic ester derivative (Example 1) | 7.0 wt% |
| 2) Glycine | 1.0 |
| 3) Sodium pyrrolidonecarboxylate | 1.0 |
| 4) Glycerol | 10.0 |
| 5) Polyoxyethylene cetyl ether | 1.5 |
| 6) Ethanol | 10.0 |
| 7) Perfume | 0.2 |
| 8) Purified water | Balance |

(Preparation)

Ingredients 1) to 8) were mixed under stirring to obtain a substantially homogeneous solution. This toilet lotion had a good compatibility to the skin and also gave moistened feel upon use.

Example 30

Lip Cream:

The tetrahydroabietic ester derivative (I) obtained in Example 1 was used for preparing a lip cream having the following composition.

(Composition)

| | |
|---|---|
| 1) Tetrahydroabietic ester derivative (Example 1) | 37.0 wt% |
| 2) Methyl-branched isostearyl glyceryl ether | 10.0 |
| 3) Jojoba oil | 16.0 |
| 4) Carnauba wax | 13.0 |
| 5) Microcrystalline wax | 14.0 |
| 6) Vaseline | 10.0 |

(Preparation)

Immediately after the ingredients 1) to 6) were mixed at 85°C under stirring for homogenization, the homogenized mixture was placed in a mold and allowed to cool down. The mixture before cooling was glossy milky-white and in a semi-solid state but after shaped into a stick form, it revealed good extendability and fittability on the skin and was not easily removed off from the lip.

Example 31

Milky Lotion:

A milky lotion having the following composition was prepared by the process below.

(Composition)

| | |
|---|---|
| 1) Silicone ester derivative (I) of Example 6 | 10.0 wt% |
| 2) Cetanol | 0.5 |
| 3) Vaseline | 1.0 |
| 4) Polyoxyethylene oleyl ether (20 E.O.) | 2.0 |
| 5) Stearic acid | 2.0 |
| 6) Glycerol | 3.0 |
| 7) Dipropylene glycol | 5.0 |
| 8) Triethanolamine | 1.0 |
| 9) Ethyl paraben | 0.1 |
| 10) Methyl paraben | 0.2 |
| 11) Perfume | Suitable amount |
| 12) Purified water | Balance |
| TOTAL | 100 |

(Process)

Ingredients 1) to 5) were allowed to dissolve at 70°C. Similarly, ingredients 6) to 12) were heated and mixed at 70°C, to which the mixture of 1) to 5) was added. The resultant mixture was emulsified by an emulsifier, followed by cooling down to 30°C by a heat exchanger. The milky lotion obtained gave moistened and thick feel upon use with reduced stickiness. It was excellent in moisture retainability as well as in sensation.

Example 32

Moisturizing Lipstick:

A lipstick having the following composition was prepared by the process below.

(Composition)

| | |
|---|---|
| 1) Silicone ester derivative (I) of Ex. 7 | 25.0 wt% |
| 2) Carnauba wax | 2.0 |
| 3) Ceresine | 4.0 |
| 4) Candelilla wax | 5.0 |
| 5) Microcrystalline wax | 2.0 |
| 6) Bees wax | 5.0 |
| 7) Lanolin | 4.0 |
| 8) Castor oil | 20.0 |
| 9) Hexadecyl alcohol | 20.0 |
| 10) Glycerol | 3.0 |
| 11) Glycerol monostearate | 2.0 |
| 12) Titanium oxide | 2.0 |
| 13) Pigment (Red #202) | 2.0 |
| 14) Pigment (Red #204) | 1.0 |
| 15) Pigment (Yellow #4 A1 lake) | 3.0 |
| 16) Antioxidant | Suitable amount |
| 17) Perfume | Suitable amount |
| TOTAL | 100 |

(Preparation)

Ingredients 1) to 7), 9) and 11) were allowed to dissolve under heat, to which ingredient 10) was dropped while stirring, followed by adding with a dispersion obtained by dispersing ingredients 12) to 17) in 8) and mixed for homogenization. The resultant mixture was placed in a mold and cooled down.

This lipstick gave a moistened feel with reduced stickiness upon use, and was excellent in moisture retainability and sensation.

Example 33

W/O-type Moisturizing Cream:

A w/o-type moisturizing cream having the following composition was prepared by the process below.

(Composition)

| | |
|---|---|
| 1) Silicone ester derivative (I) of Ex. 8 | 45.0 wt% |
| 2) Paraffin | 5.0 |
| 3) Sorbitan sesquioleate | 3.0 |
| 4) Aluminum stearate | 0.5 |
| 5) Magnesium disulfide | 1.0 |
| 6) Polyethylene glycol 1500 | 2.0 |
| 7) Glycerol | 3.0 |
| 8) Polyoxyethylene methyl glucoside | 3.0 |
| 9) Perfume | Suitable amount |
| 10) Antioxidant | Suitable amount |
| 11) Purified water | Balance |
| TOTAL | 100 |

(Process)

Ingredients 5) to 8), 10) and 11) were mixed at 70°C. Similarly, ingredients 1) to 4) were mixed at 70°C, to which the mixture of 5) to 8), 10) and 11) was added. The resultant mixture was emulsified by an emulsifier, added with 9) and stirred for homogenization. The resultant emulsion was cooled down to 30°C by a heat exchanger to obtain a w/o moisturizing cream. The cream obtained gave moistened and thick feel upon use with reduced stickiness. It was excellent in moisture-retaining ability as well as feel on use.

Example 34

Hand Cream:

The silicone tetrahydroabietic ester derivative obtained in Example 9 was used to prepare an emulsion having the following composition.

(Composition)

| | |
|---|---|
| 1) Silicone tetrahydroabietic ester derivative of Ex. 9 | 5.0 wt% |
| 2) Stearic acid | 10.0 |
| 3) Stearic acid monoglyceride | 1.5 |
| 4) Polyoxyethylene monostearate | 1.5 |

Continuation of the Table on the next page

(continued)

| | |
|---|---|
| 5) Triethanolamine | 0.3 |
| 6) Methyl paraben | 0.1 |
| 7) Butyl paraben | 0.1 |
| 8) Perfume | 0.2 |
| 9) Purified water | Balance |

(Process)

Ingredients 1) to 4) were mixed at 70'C, to which a 70°C mixture of 5) to 7) and 9) was added under stirring for effecting emulsification. The resultant mixture was cooled down to 40°C, to which ingredient 8) was added to obtain a white emulsion. This emulsion had good fittability on the skin, and was hard to be removed by hand-washing, revealing excellent properties as a hand cream.

Example 35

Facial Pack:

The silicone tetrahydroabietic ester derivative obtained in Example 9 was used to prepare a mixture having the following composition.

(Composition)

| | |
|---|---|
| 1) Silicone tetrahydroabietic ester derivative of Ex. 9 | 2.5 wt% |
| 2) Polyvinyl alcohol | 15.0 |
| 3) Titanium oxide | 5.0 |
| 4) Ethylene glycol | 4.0 |
| 5) Methyl paraben | 0.1 |
| 6) Perfume | 0.2 |
| 7) Purified water | Balance |

(Process)

Ingredient 7) was heated at about 90°C, to which ingredient 2) was added under stirring by small amounts and allowed to dissolve until homogeneous solution was obtained. Ingredients 1) and 3) to 5) were further added thereto and stirred for homogenization. After cooling down to about 40°C, ingredient 6) was added and mixed. The obtained mixture, when used as a facial pack, gave moderate stimulation to the skin when peeled off from the facial skin and gave a prolonged moisturized feeling.

Example 36

Lip Cream:

The silicone tetrahydroabietic ester derivative obtained in Example 9 was used to prepare a mixture having the following composition.

(Composition)

| | |
|---|---|
| 1) Siliocone tetrahydroabietic ester derivative of Ex. 9 | 10.0 wt% |

Continuation of the Table on the next page

(continued)

| | |
|---|---|
| 2) Liquid paraffin | 37.0 |
| 3) Jojoba oil | 16.0 |
| 4) Carnauba wax | 13.0 |
| 5) Microcrystalline wax | 14.0 |
| 6) Vaseline | 10.0 |

(Process)

Immediately after the ingredients 1) to 6) were mixed at 85°C under stirring for homogenization, the homogenized mixture was placed in a mold and allowed to cool down. The mixture before cooling was glossy milky-white and in a semi-solid state but after shaped in to a stick form, it revealed good extendability and fittability on the skin and was not easily removed off from the lip.

Example 37

Lotion:

The silicone tetrahydroabietic ester derivative obtained in Example 9 was used to prepare a mixture having the following composition.

(Composition)

| | |
|---|---|
| 1) Silicone tetrahydroabietic ester derivative of Ex. 9 | 5.0 wt% |
| 2) Ethanol | 15.0 |
| 3) L-Serine | 1.0 |
| 4) Sodium pyrrolidone carboxylate | 1.0 |
| 5) Polyoxyethylene oleyl ether | 1.5 |
| 6) Perfume | 0.2 |
| 7) Purified water | Balance |

(Process)

Ingredients 1) to 7) were mixed under stirring for homogenization. The thus obtained lotion revealed a good fittability on the skin and gave moistened feel on use.

Example 38

Moisturizing Lotion:

A moisturizing lotion having the following composition was prepared by the process below.

| | |
|---|---|
| 1) Silicone ester derivative (I) of Ex. 13 | 1.0 wt% |
| 2) Polyoxyethylene oleyl ether (20 E.O.) | 1.5 |
| 3) Glycerol | 5.0 |
| 4) 1,3-Butanediol | 5.0 |
| 5) Polyethlene glycol 1500 | 1.5 |

| | |
|---|---|
| 6) Ethanol | 10.0 |
| 7) Methyl paraben | 0.1 |
| 8) Perfume | Suitable amount |
| 9) Citric acid | Suitable amount |
| 10) Sodium citrate | " " |
| 11) Purified water | Balance |
| TOTAL | 100.0 |

(Process)

Ingredients 1), 2), 6) and 8) were mixed well, to which mixture of 3) to 5), 7) and 9) to 11) was added and stirred to obtain a substantially homogeneous solution. This moisturizing lotion was capable of giving moistened and thick feel upon use with reduced stickiness and was excellent in moisturizing effect and sensation.

Example 39

Milky Lotion:

A milky lotion having the following composition was prepared by the process below.

(Composition)

| | |
|---|---|
| 1) Silicone ester derivative (I) of Ex.14 | 10.0 wt% |
| 2) Cetanol | 0.5 |
| 3) Vaseline | 1.0 |
| 4) Polyoxyethylene oleylether (20 E.O.) | 2.0 |

| | |
|---|---|
| 5) Stearic acid | 2.0 |
| 6) Glycerol | 3.0 |
| 7) Dipropylene glycol | 5.0 |
| 8) Triethanolamine | 1.0 |
| 9) Ethyl paraben | 0.1 |
| 10) Methyl paraben | 0.2 |
| 11) Perfume | Suitable amount |
| 12) Purified water | Balance |
| TOTAL | 100.0 |

(Process)

Ingredients 1) to 5) were allowed to dissolve under heat, and the temperature was maintained at 70°C. Ingredients 6) to 10) and 12) were also mixed at 70°C, to which the mixture of 1) to 5) was added. The obtained mixture was charged in an emulsifier for emulsification. The resultant emulsion was cooled down to 40°C and added with ingredient 11) and mixed. A heat exchanger was used to cool down the resultant mixture to 30°C to obtain a milky lotion.

This milky lotion gave moistened and thick feel upon use with reduced stickiness. It was excellent in moisture-retaining ability and feel on use.

Example 40

Moisturizing Cream:

A moisturizing cream having the following composition was prepared by the process below.

(composition)

| | |
|---|---|
| 1) Silicone ester derivative (I) of Ex. 15 | 8.0 wt% |
| 2) Silicone ester derivative (I) of Ex. 16 | 2.0 |
| 3) Paraffin | 2.0 |
| 4) Cetyl 2-ethylhexanoate | 5.0 |
| 5) Lanolin | 5.0 |
| 6) Bees wax | 2.0 |
| 7) Stearyl alcohol | 4.0 |
| 8) Self-emulsified glyceryl monostearate | 1.5 |
| 9) Polyoxyethylene sorbitan monooleate (20 E.O.) | 1.0 |
| 10) Glycerol | 5.0 |
| 11) 70% Sorbitol | 10.0 |
| 12) Ethyl paraben | Suitable amount |
| 13) Methyl paraben | Suitable amount |

Continuation of the Table on the next page

(continued)

| | |
|---|---|
| 14) Perfume | Suitable amount |
| 15) Purified water | Balance |
| TOTAL | 100.0 |

(Process)

Ingredients 1) to 9) were allowed to dissolve under heat, and the temperature was maintained at 70°C. In the same manner, ingredients 10) to 13) and 15) were heated at 70°C and mixed, to which the mixture of 1) to 9) was added. The obtained mixture was charged in an emulsifier for emulsification. The resultant emulsion was cooled down to 40°C under stirring, to which ingredient 14) was further added and mixed well. A heat exchanger was used to cool down to 30°C to obtain a moisturizing cream.

This moisturizing cream was excellent in moisture retainability and feel on use, giving moistened feel with reduced stickiness.

Example 41

Cold Cream:

A cold cream having the following composition was prepared by the process below.

(Composition)

| | |
|---|---|
| 1) Silicone ester derivative (I) of Ex.17 | 28.0 wt% |
| 2) Silicone ester derivative (I) of Ex.18 | 2.0 |
| 3) Bees wax | 5.0 |
| 4) Spermaceti | 3.0 |
| 5) Cetyl 2-ethylhaxnoate | 10.0 |
| 6) Cetanol | 1.0 |
| 7) Self-emulsified glyceryl monostearate | 7.0 |
| 8) Polyoxyethylene sorbitan monooleate (20 E.O.) | 2.0 |
| 9) Glycerol | 5.0 |
| 10) Triethanolamine | 0.3 |

| | |
|---|---|
| 11) Ethyl paraben | Suitable amount |
| 12) Methyl paraben | " " |
| 13) Perfume | " " |
| 14) Purified water | Balance |
| TOTAL | 100.0 |

(Process)

Ingredients 1) to 8) were allowed to dissolve under heat, and temperature was maintained at 70°C. In the same manner, ingredients 9) to 12) and 14) were heated at 70°C and mixed, to which the mixture of 1) to 8) was slowly added. The obtained mixture was charged in an emulsifier for emulsification. The resultant emulsion was cooled down to 40°C under stirring to which ingredient 13) was further added and mixed well. A heat exchanger was used to cool down to 30°C to obtain a cold cream.

This cold cream was excellent in moisture retainability and feel on use giving thick and moistened feel with reduced stickiness.

Example 42

Creamy Moisturizing Foundation:

A creamy moisturizing foundation having the following composition was prepared by the process below.

(Composition)

| | |
|---|---|
| 1) Silicone ester derivative (I) of Ex. 19 | 10.0 wt% |
| 2) Liquid paraffin | 8.0 |
| 3) Squalane | 8.0 |
| 4) Neopentyl glycol dioctanoate | 3.0 |
| 5) Sorbitan sesqui isostearate | 7.0 |
| 6) Aluminum distearate | 0.2 |
| 7) Magnesium sulfate | 0.7 |
| 8) Martitol | 2.0 |
| 9) Glycerol | 3.0 |
| 10) Methyl paraben | 0.1 |
| 11) Titanium oxide | 8.0 |
| 12) Talc | 5.0 |
| 13) Sericite | 2.0 |
| 14) Red iron oxide | 0.4 |
| 15) Iron (III) oxide | 0.7 |
| 16) Iron (II) oxide | 0.1 |
| 17) Perfume | Suitable amount |
| 18) Purified water | Balance |
| TOTAL | 100.0 |

(Process)

Ingredients 1) to 6) were heated at 70°C and mixed well. In this homogeneous mixture, ingredients 11) to 16) were dispersed, to which a homogeneous mixture of ingredients 7) to 10) and 18) heated at 70°C were added under stirring for effecting emulsification. The emulsion was cooled down to 40°C under stirring, when 17) was further added and cooled to room temperature under stirring.

This creamy foundation gave moistened feel with reduced stickiness, and was excellent in moisture retainability and sensation.

Example 43

Moisturizing Lipstick:

A moisturizing lipstick having the following composition was prepared by the process below.

(Composition)

| | |
|---|---|
| 1) Silicone ester derivative (I) of Ex.20 | 20.0 wt% |
| 2) Silicone ester derivative (I) of Ex.21 | 5.0 |
| 3) Carnauba wax | 2.0 |
| 4) Ceresine | 4.0 |
| 5) Candelilla wax | 5.0 |
| 6) Microcrystalline wax | 2.0 |
| 7) Bees wax | 5.0 |
| 8) Lanolin | 4.0 |
| 9) Castor oil | 20.0 |
| 10) Hexadecyl alcohol | 20.0 |
| 11) Glycerol | 3.0 |
| 12) Glycerol monostearate | 2.0 |

| | | |
|---|---|---|
| 13) | Titanium oxide | 2.0 |
| 14) | Pigment (Red #202) | 2.0 |
| 15) | Pigment (Red #204) | 1.0 |
| 16) | Pigment (Yellow #4 Al lake) | 3.0 |
| 17) | Antioxidant | Suitable amount |
| 18) | Perfume | " " |
| | TOTAL | 100.0 |

(Process)

Ingredients 1) to 8), 10) and 12) were allowed to dissolve under heat, to which ingredient 11) was dropped while stirring, followed by adding with a dispersion obtained by dispersing ingredients 13) to 18) in 9) and mixed for homogenization. The resultant mixture was placed in a mold and cooled down.

This lipstick gave a moistened feel with reduced stickiness upon use, and was excellent in moisture retainability and sensation.

Example 44

Nail Enamel Remover:

A nail enamel remover having the following composition was prepared by the process below.

(Composition)

| | | |
|---|---|---|
| 1) | Acetone | 60.0 wt% |
| 2) | Butyl acetate | 30.0 |
| 3) | 1,3-Butanediol | 2.0 |
| 4) | Silicone ester derivative (I) of Ex.21 | 1.0 |
| 5) | Dye | Suitable amount |
| 6) | Perfume | " " |
| 7) | Purified water | Balance |
| | TOTAL | 100.0 |

(Process)

Ingredients 1) to 7) were homogeneously mixed to prepare a nail enamel remover. This remover was excellent in feel on use.

## Claims

### Claims for the following Contracting States : DE, FR

1. An alcohol-modified silicone ester derivative of the following formula (I):

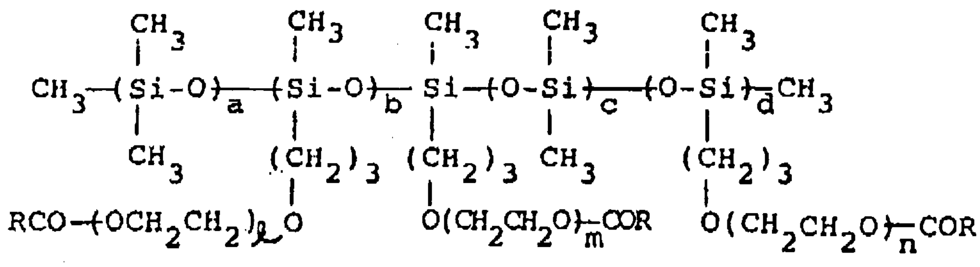

(I)

wherein the group RCO- is selected from abietic acid residue, dehydroabietic acid residue, dihydroabietic acid residue, tetrahydroabietic acid residue and cholic acid residue; a, b, and c are numbers which sum falls in the range of 0 to 130; d is 0 or 1, provided that the case a = b = c = d = 0 is excluded; and 1, m, and n are numbers which sum falls in the range from 0 to 6.

2. The alcohol-modified silicone ester derivative according to Claim 1, wherein each of a, b, and c is 0.

3. A cosmetic composition comprising an alcohol-modified silicone ester derivative as defined in Claim 1.

4. A cosmetic composition according to Claim 3, further comprising a polyol-type moisturizer.

### Claims for the following Contracting State : ES

1. A process for preparing an alcohol-modified silicone ester derivative of the following formula (I):

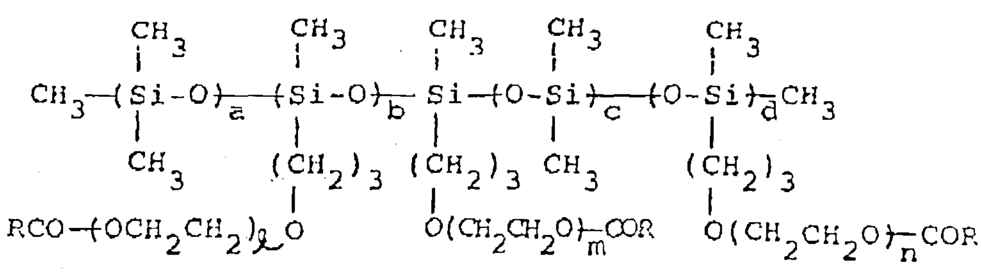

(I)

wherein the group RCO- is selected from abietic acid residue, dehydroabietic acid residue, dihydroabietic acid residue, tetrahydroabietic acid residue and cholic acid residue; a, b, and c are numbers which sum falls in the range of 0 to 130; d is 0 or 1, provided that the case a = b = c = d = 0 is excluded; and 1, m, and n are numbers which sum falls in the range from 0 to 6, which comprises reacting an alcohol-modified silicone (II) with a carboxylic acid (III) or its reactive derivative according to the following reaction scheme,

<u>Process A:</u>

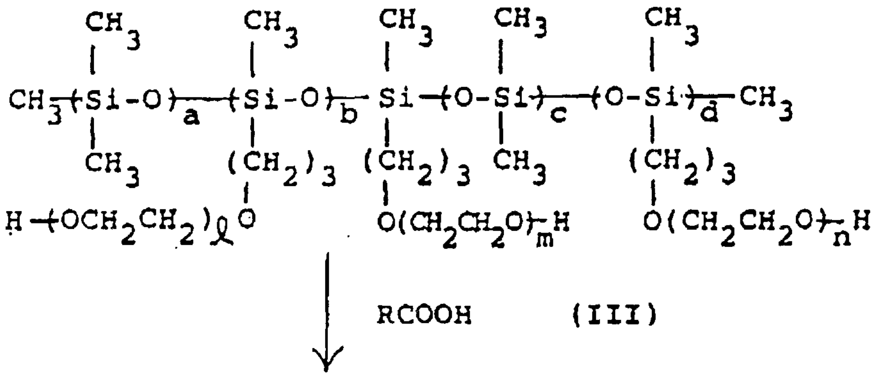


(II)

(I)

wherein R, a, b, c, l, m and n have the same meanings as defined before; or

a polysiloxane (IV) having Si-H bonds and a carboxylic acid ester (V) are reacted for hydrosilylation at room temperature or under heat in the presence of a catalytic amount of chloroplatinic acid catalyst according to the following reaction scheme

Process B

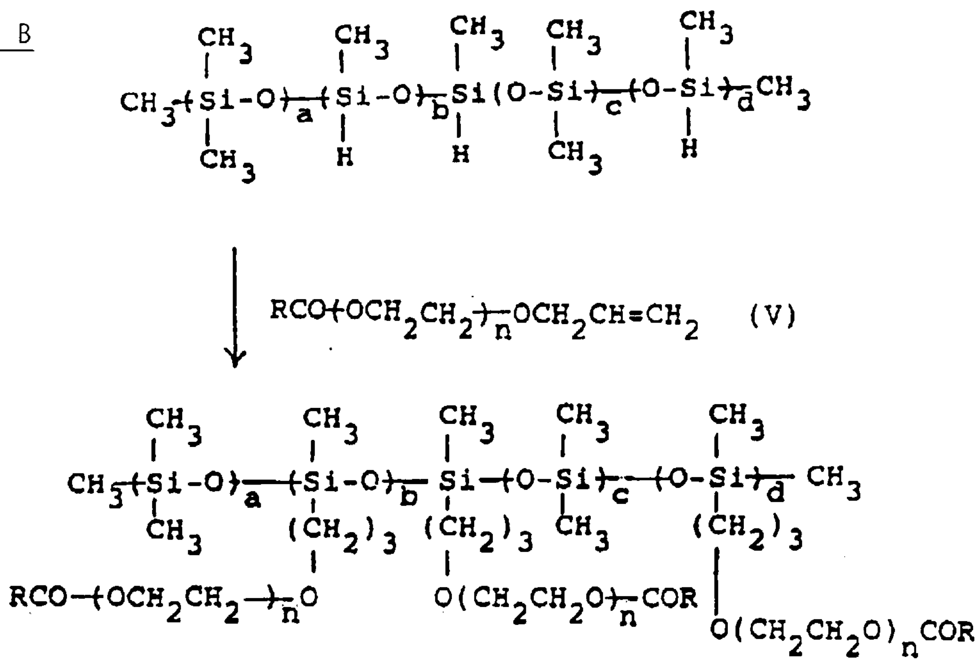


(IV)

(VI)

wherein R, a, b, c, d and n have the same meanings as defined before.

2. A process according to claim 1, wherein each of a, b and c is 0.

3. A cosmetic composition comprising an alcohol-modified silicone ester derivative of formula (I) as defined in claim 1 in an amount from 0.001 to 90 wt% of the total composition.

4. A cosmetic composition according to claim 3, further comprising a polyol-type moisturizer.

## Patentansprüche

### Patentansprüche für folgende Vertragsstaaten : DE, FR

**1.** Alkohol-modifiziertes Siliconester-Derivat der folgenden Formel (1):

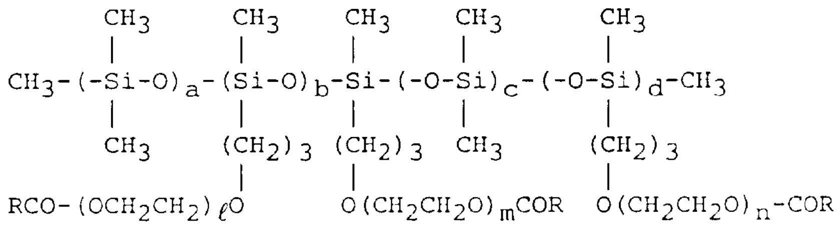

(I)

worin die Gruppe RCO- ausgewählt ist aus dem Rest der Abietinsäure, Rest der Dehydroabieinsäure, Rest der Dihydroabietinsäure, Rest der Tetrahydroabietinsäure und Rest der Cholsäure; a, b und c Zahlen sind, deren Summe in den Bereich von 0 bis 130 fällt, d 0 oder 1 ist, unter der Bedingung, daß der Fall a = b = c = d = 0 ausgeschlossen ist, und $\ell$, m und n Zahlen sind, deren Summe in den Bereich von 0 bis 6 fällt.

**2.** Alkohol-modifiziertes Siliconester-Derivat nach Anspruch 1, worin jedes von a, b und c 0 ist.

**3.** Kosmetische Zusammensetzung, umfassend ein alkohol-modifiziertes Siliconester-Derivat nach Anspruch 1.

**4.** Kosmetikzusammensetzung nach Anspruch 3, die weiter ein Feuchtigkeitsmittel vom Polyoltyp umfaßt.

### Patentansprüche für folgenden Vertragsstaat : ES

**1.** Verfahren zum Herstellen eines alkohol-modifizierten Siliconester-Derivats der folgenden Formel (I):

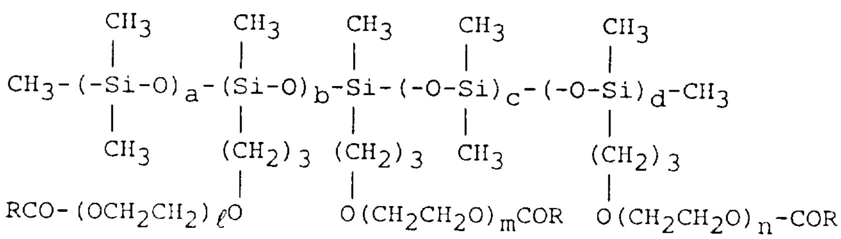

(I)

worin die Gruppe RCO- ausgewählt ist aus dem Rest der Abietinsäure, Rest der Dehydroabietinsäure, Rest der Dihydroabietinsäure, Rest der Tetrahydroabietinsäure und Rest der Cholsäure; a, b und c Zahlen sind, deren Summe in den Bereich von 0 bis 130 fällt, d 0 oder 1 ist, unter der Bedingung, daß der Fall a = b = c = d = 0 ausgeschlossen ist, und $\ell$, m und n Zahlen sind, deren Summe in den Bereich von 0 bis 6 fällt,
umfassend das Umsetzen eines Alkohol-modifizierten Silicons (II) mit einer Carbonsäure (III) oder deren reaktionsfähigem Derivat gemäß dem folgenden Reaktionsschema
<u>Verfahren A:</u>

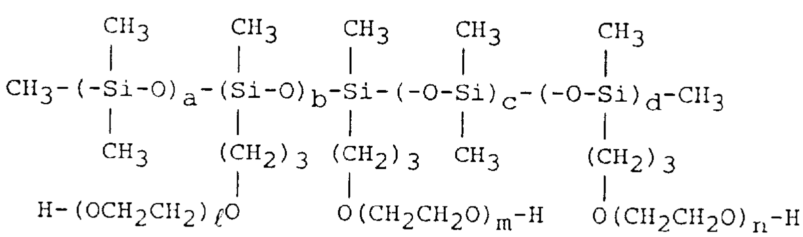

(II)

RCOOH (III)

(I)

worin R, a, b, c, ℓ, m und n die oben genannten Bedeutungen haben oder Umsetzen eines Polysiloxans (IV) mit Si-H-bindungen und eines Carbonsäureesters (V) durch Hydrosilylierung bei Raumtemperatur oder unter Erwärmen in Gegenwart einer kataytischen Menge von Chlorplatinsäure-Katalysator gemäß dem folgenden Reaktionsschema Verfahren B:

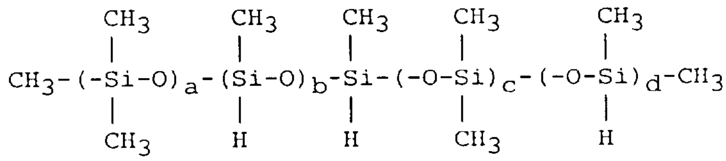

(IV)

$RCO-(OCH_2CH_2)_n-OCH_2CH=CH_2$ (V)

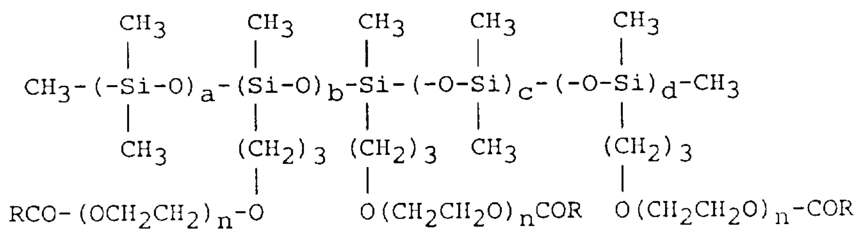

(VI)

worin R, a, b, c, d und n die oben angegebenen Bedeutungen haben.

**2.** Verfahren Anspruch 1, worin jedes von a, b und c 0 ist.

**3.** Kosmetische Zusammensetzung, umfassend ein alkohol-modifiziertes Siliconester-Derivat der Formel (I) nach Anspruch 1 in einer Menge von 0,001 bis 90 Gew.-% der Gesamtzusammensetzung.

**4.** Kosmetikzusammensetzung nach Anspruch 3, die weiter ein Feuchtigkeitsmittel vom Polyoltyp umfaßt.

## Revendications

### Revendications pour les Etats contractants suivants : DE, FR

**1.** Dérivé d'ester de silicone modifié par alcool ayant la formule (I) suivante :

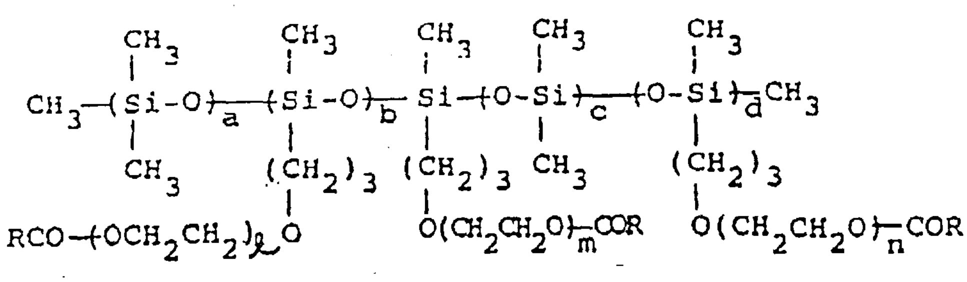

(I)

dans laquelle :

- le groupe RCO- est choisi parmi un reste d'acide abiétique, un reste d'acide déhydroabiétique, un reste d'acide dihydroabiétique, un reste d'acide tétrahydroabiétique et un reste d'acide cholique ;
- a, b et c sont des nombres dont la somme est située dans la plage de 0 à 130 ;
- d représente 0 ou 1, à condition que le cas a = b = c = d = 0 soit exclu ; et
- 1, m et n sont des nombres dont la somme est située dans la plage de 0 à 6.

**2.** Dérivé d'ester de silicone modifié par alcool, selon la revendication 1, dans lequel a, b et c représentent chacun 0.

**3.** Composition cosmétique comprenant un dérivé d'ester de silicone modifié par alcool tel que défini à la revendication 1.

**4.** Composition cosmétique selon la revendication 3, comprenant en outre un agent hydratant de type polyol.

**Revendications pour l'Etat contractant suivant : ES**

**1.** Procédé de préparation d'un dérivé d'ester de silicone modifié par alcool ayant la formule (I) suivante :

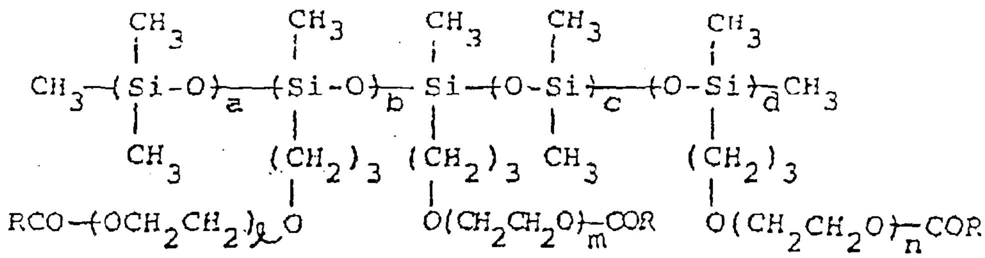

(I)

dans laquelle :

- le groupe RCO- est choisi dans le groupe constitué par un reste d'acide abiétique, un reste d'acide déhydroabiétique, un reste d'acide dihydroabiétique, un reste d'acide tétrahydroabiétique et un reste d'acide cholique ;
- a, b et c sont des nombres dont la somme est située dans la plage de 0 à 130 ;
- d représente 0 ou 1, à condition que le cas a = b = c = d = 0 soit exclu ; et
- 1, m et n sont des nombres dont la somme est située dans la plage de 0 à 6,

lequel procédé comprend la réaction d'un silicone modifié par alcool (II) avec un acide carboxylique (III) ou son dérivé réactif, conformément au schéma réactionnel suivant,
<u>Procédé A</u> :

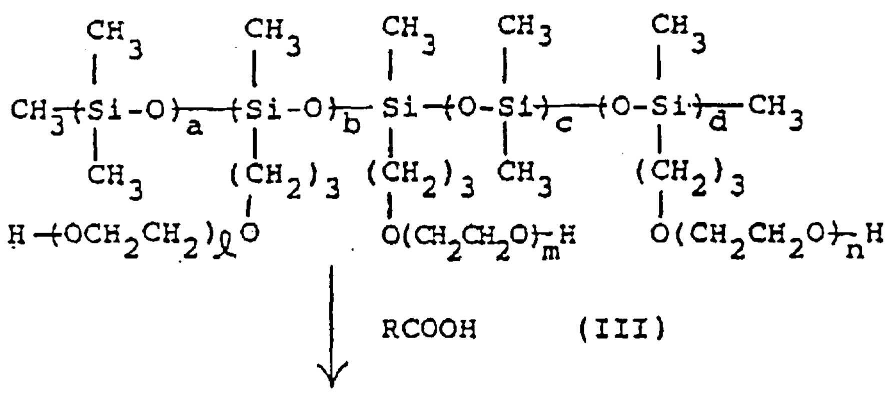


(II)

(I)

dans lequel R, a, b, c, l, m et n ont les mêmes significations que celles définies auparavant ; ou un polysiloxane (IV) ayant des liaisons Si-H et un ester d'acide carboxylique (V) sont mis à réagir en vue d'une hydrosilylation à la température ambiante ou sous chauffage en présence d'une quantité catalytique de catalyseur acide chloroplatinique conformément au schéma réactionnel suivant :

Procédé B

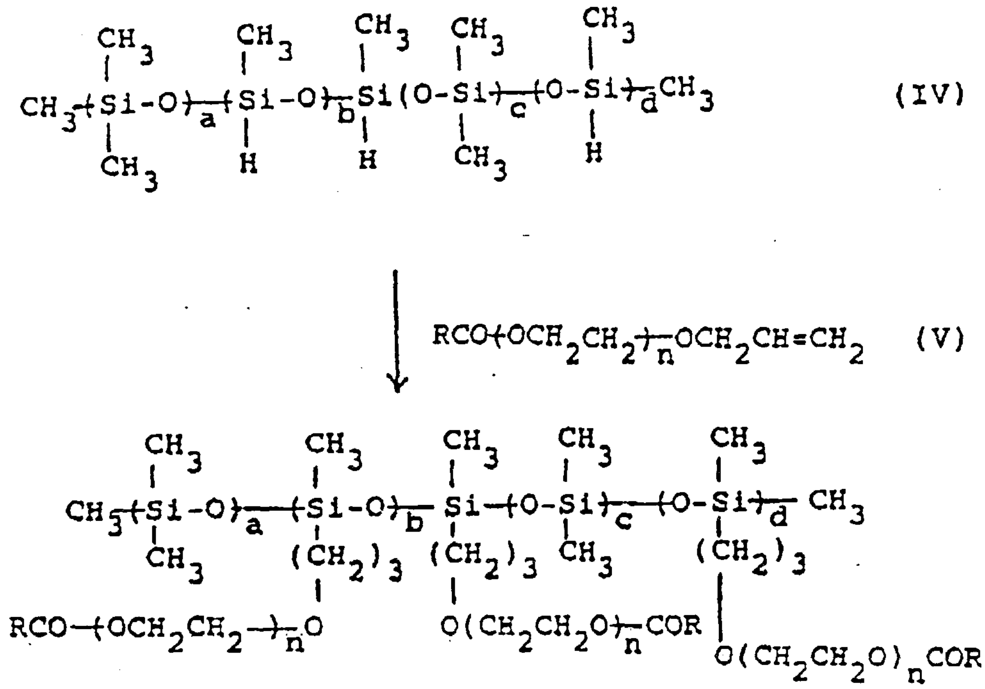

(IV)

(V)

dans lequel R, a, b, c, d et n ont les mêmes significations que celles définies auparavant.

**2.** Procédé selon la revendication 1, dans lequel a, b et c représentent chacun 0.

**3.** Composition cosmétique comprenant un dérivé d'ester de silicone modifié par alcool de formule (I) tel que défini à la revendication 1 dans une quantité de 0,001 à 90% en poids de la composition totale.

**4.** Composition cosmétique selon la revendication 3, comprenant en outre un agent hydratant de type polyol.

FIG. 1

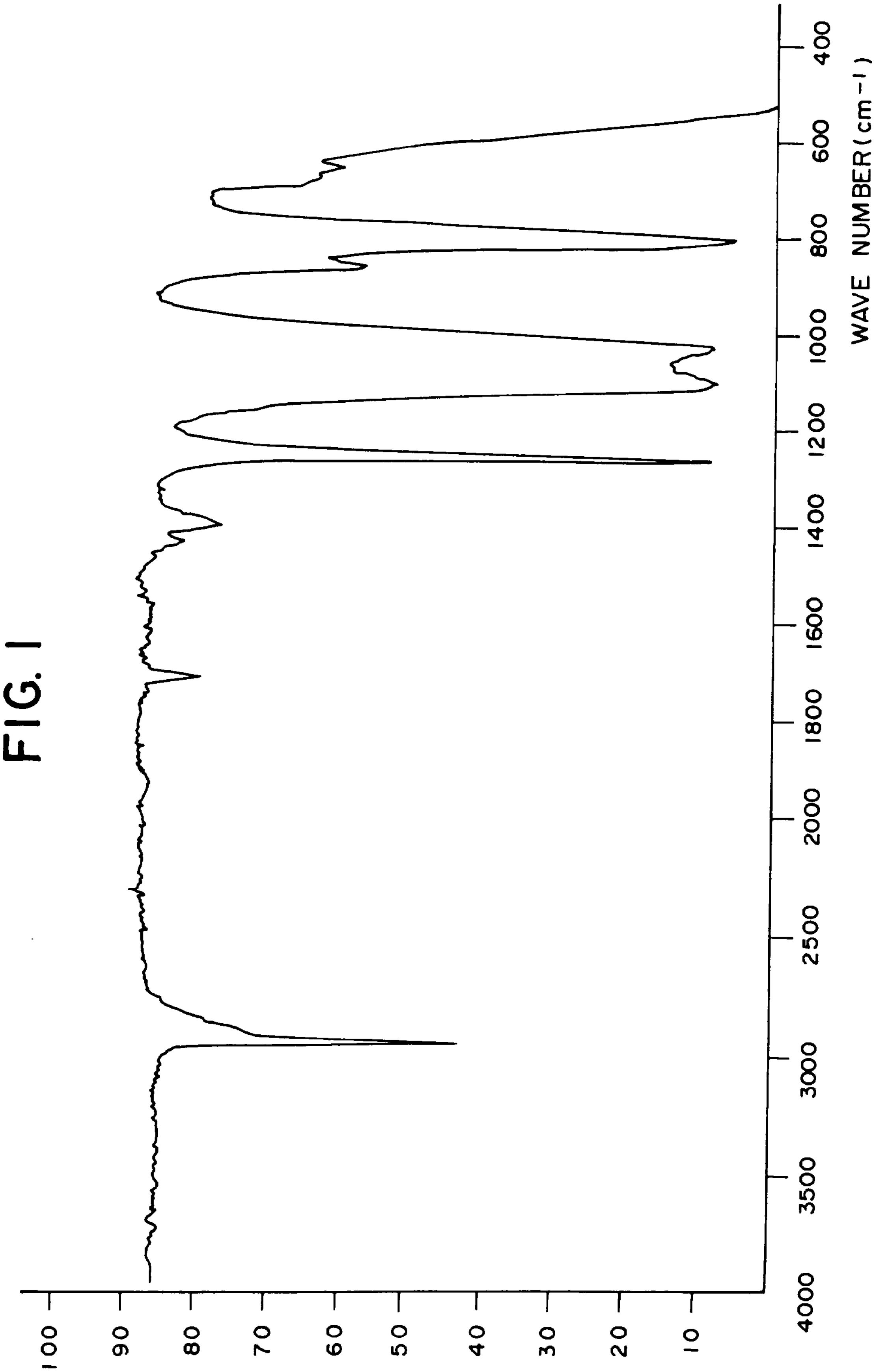
100
90
80
70
60
50
40
30
20
10
4000
3500
3000
2500
2000
1800
1600
1400
1200
1000
800
600
400
WAVE NUMBER (cm⁻¹)

FIG. 2

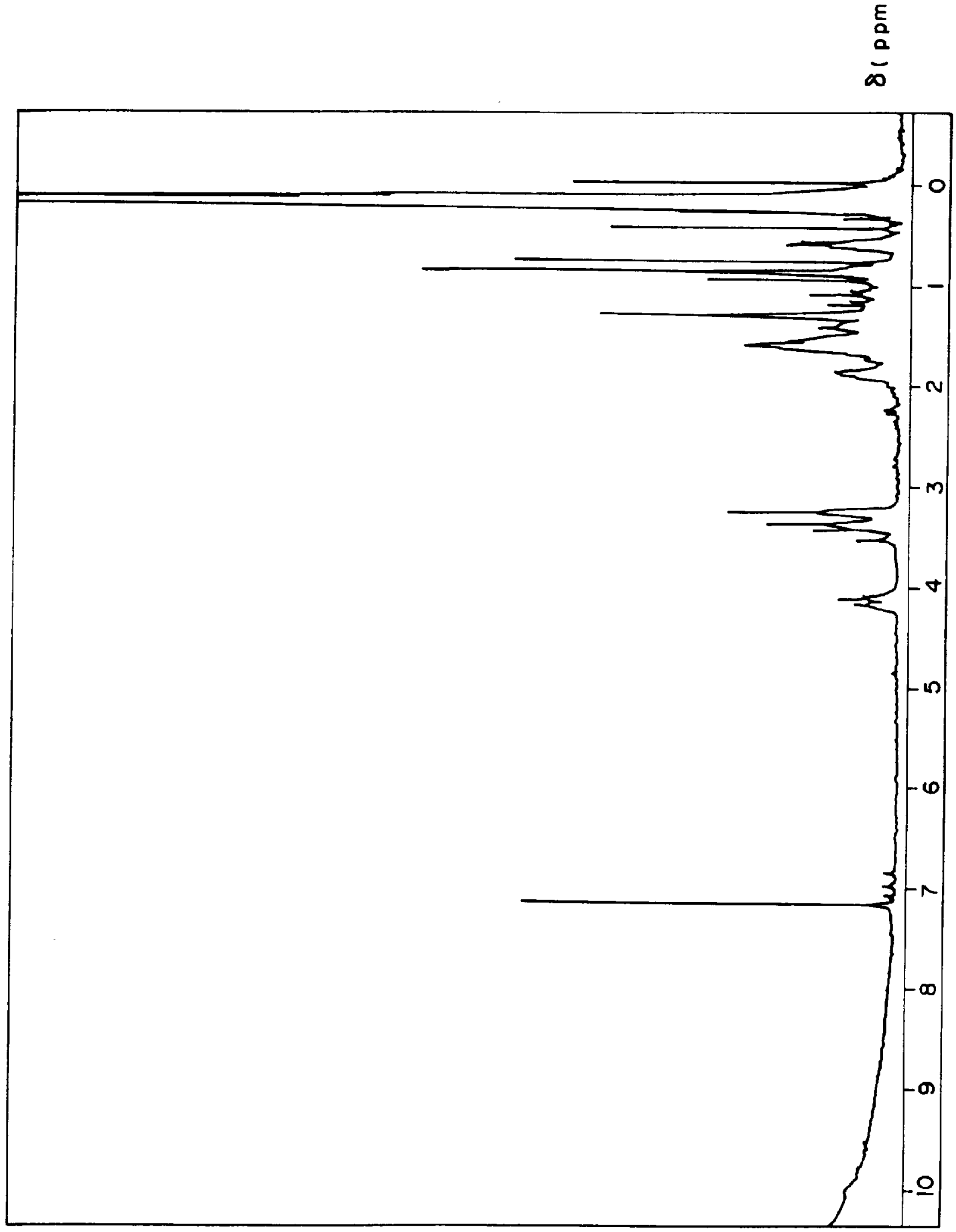

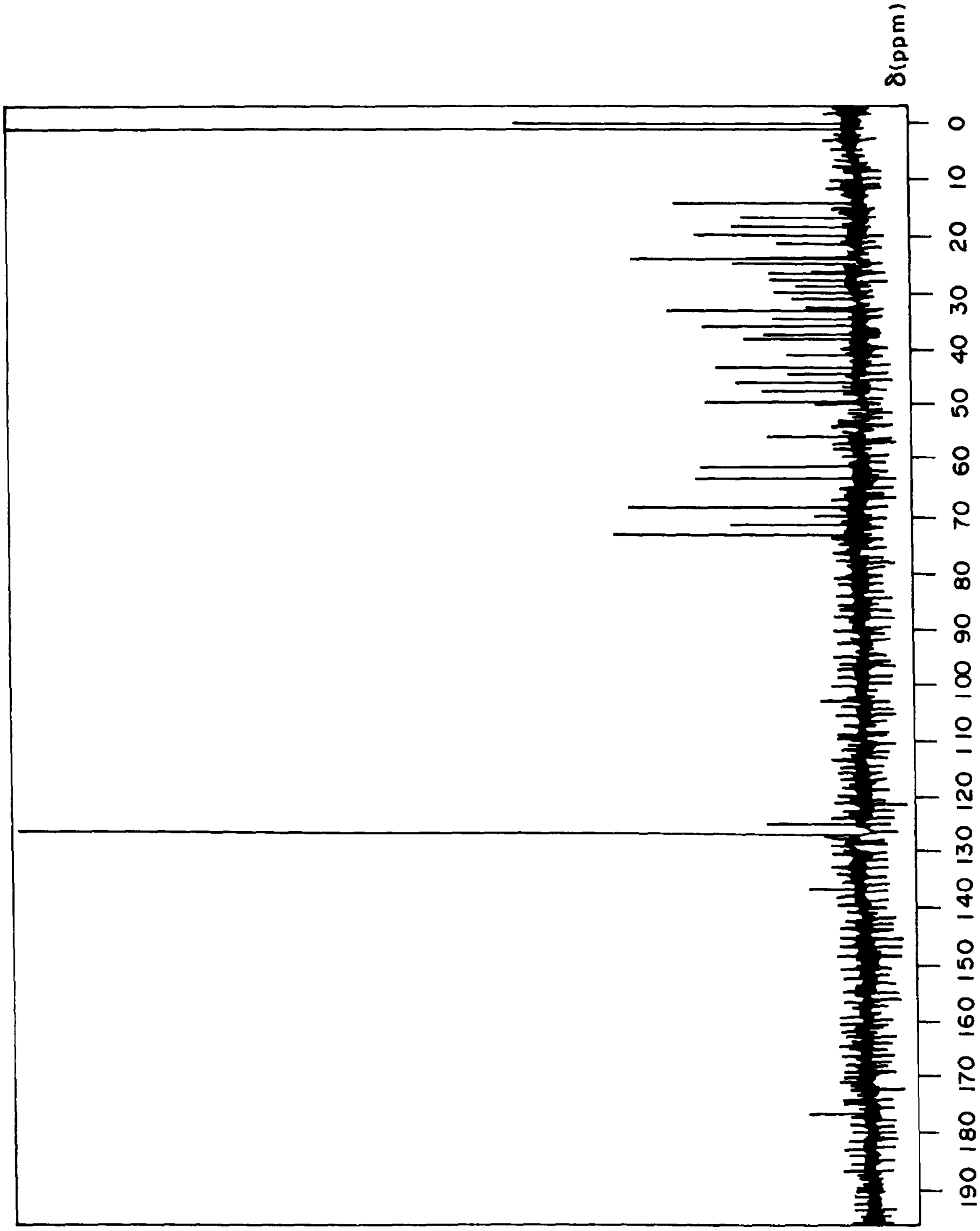
δ(ppm)
0
10
20
30
40
50
60
70
80
90
100
110
120
130
140
150
160
170
180
190

FIG. 3

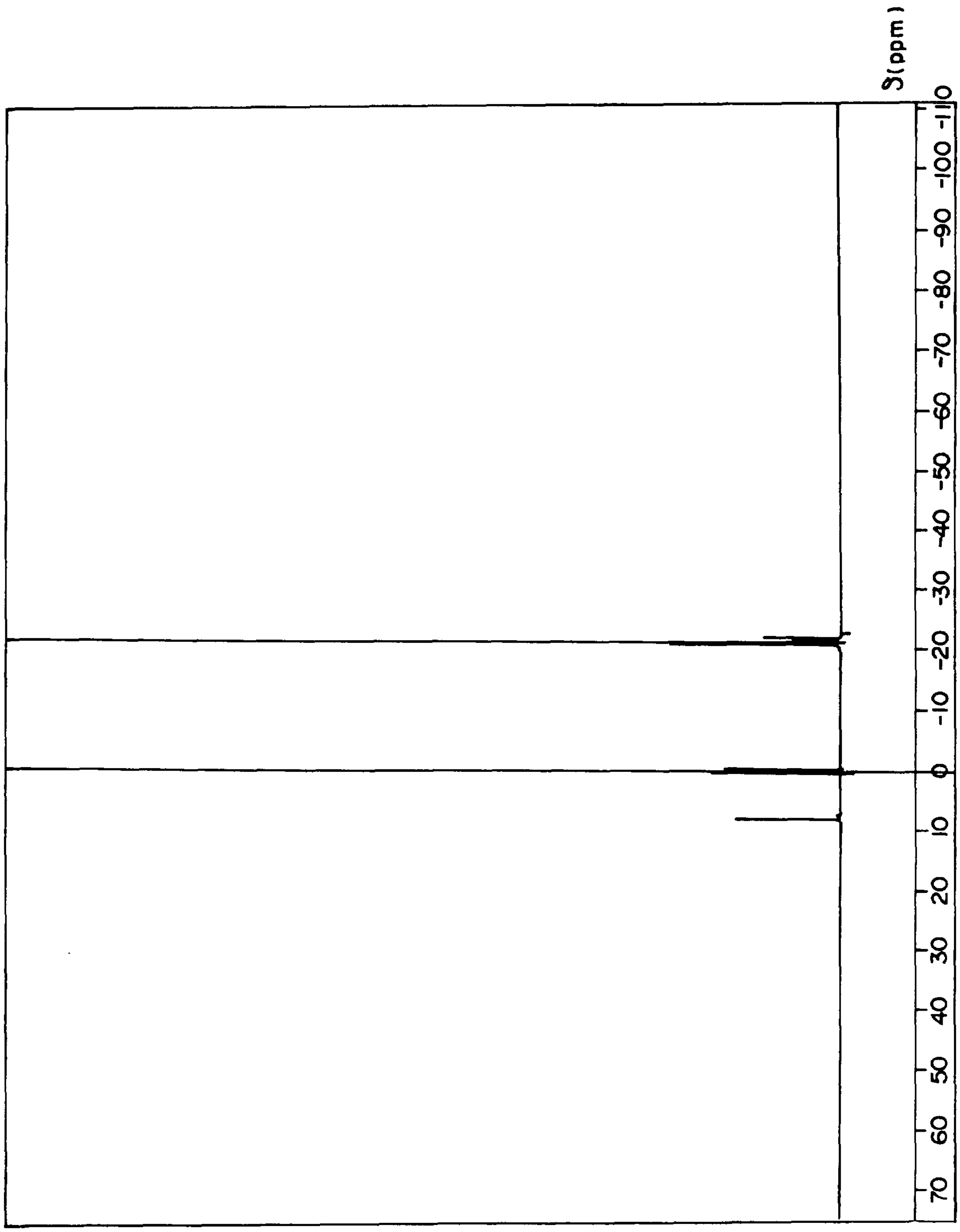


FIG. 4

FIG. 5

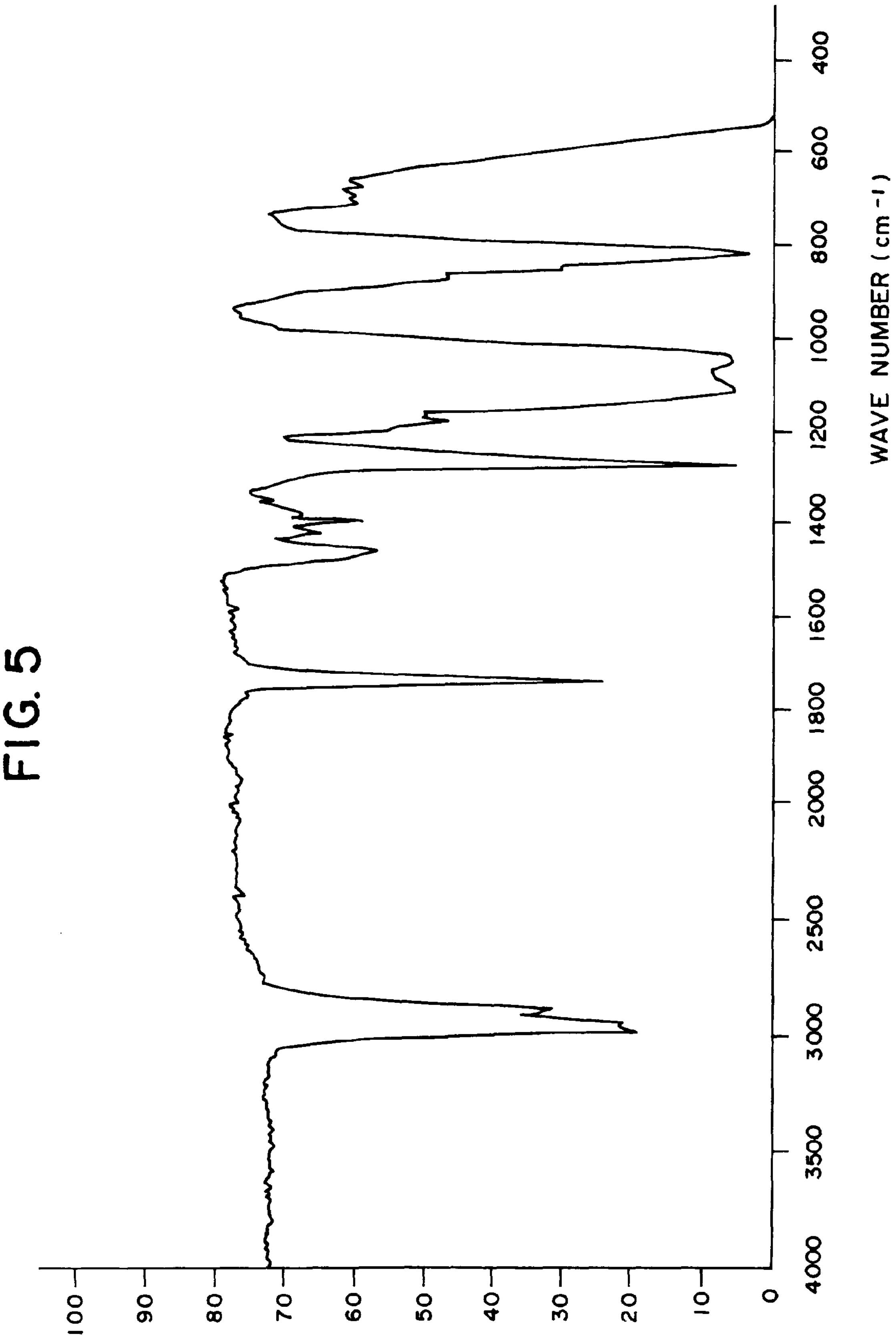
WAVE NUMBER (cm -1)
4000 3500 3000 2500 2000 1800 1600 1400 1200 1000 800 600 400
0 10 20 30 40 50 60 70 80 90 100

FIG. 6

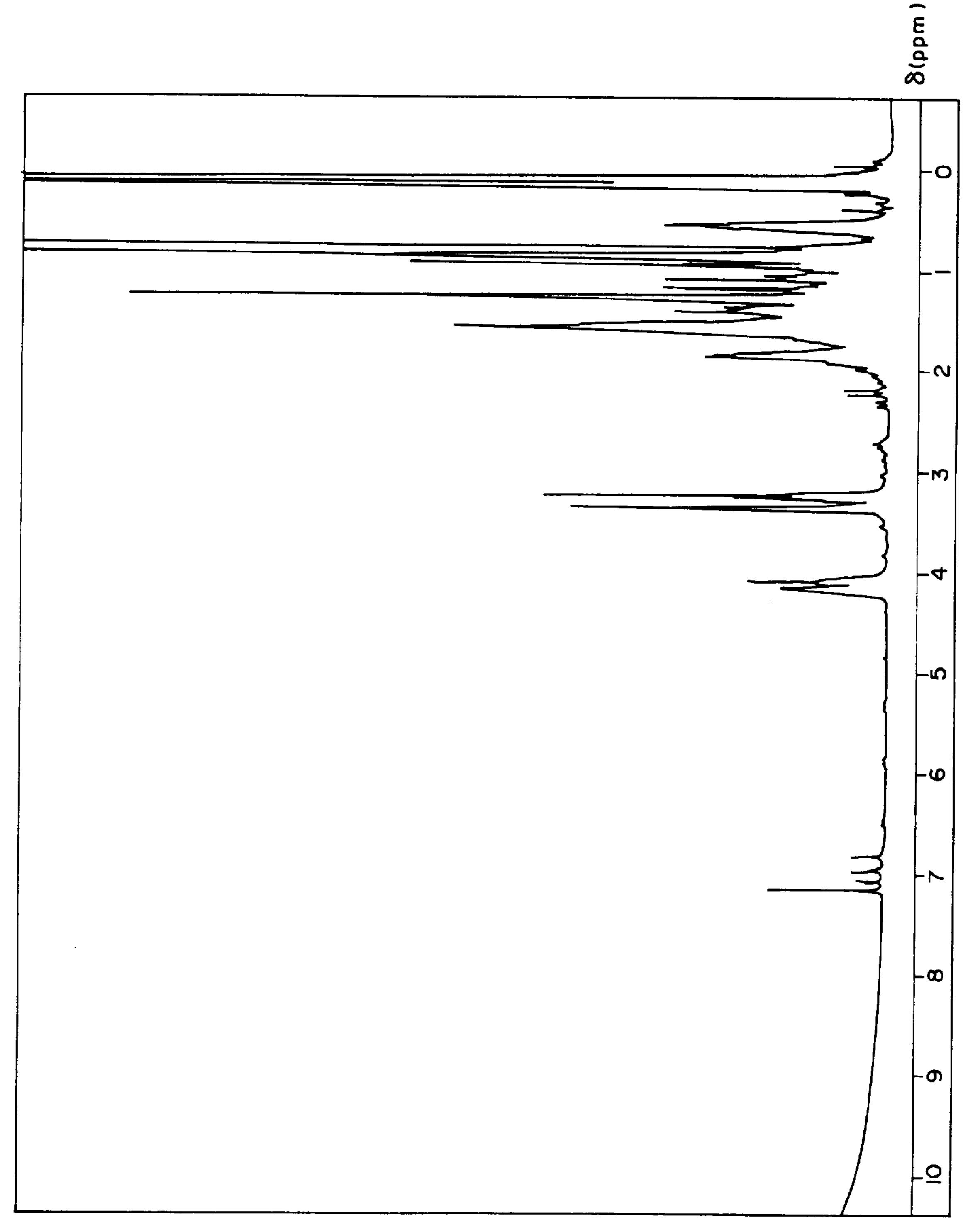

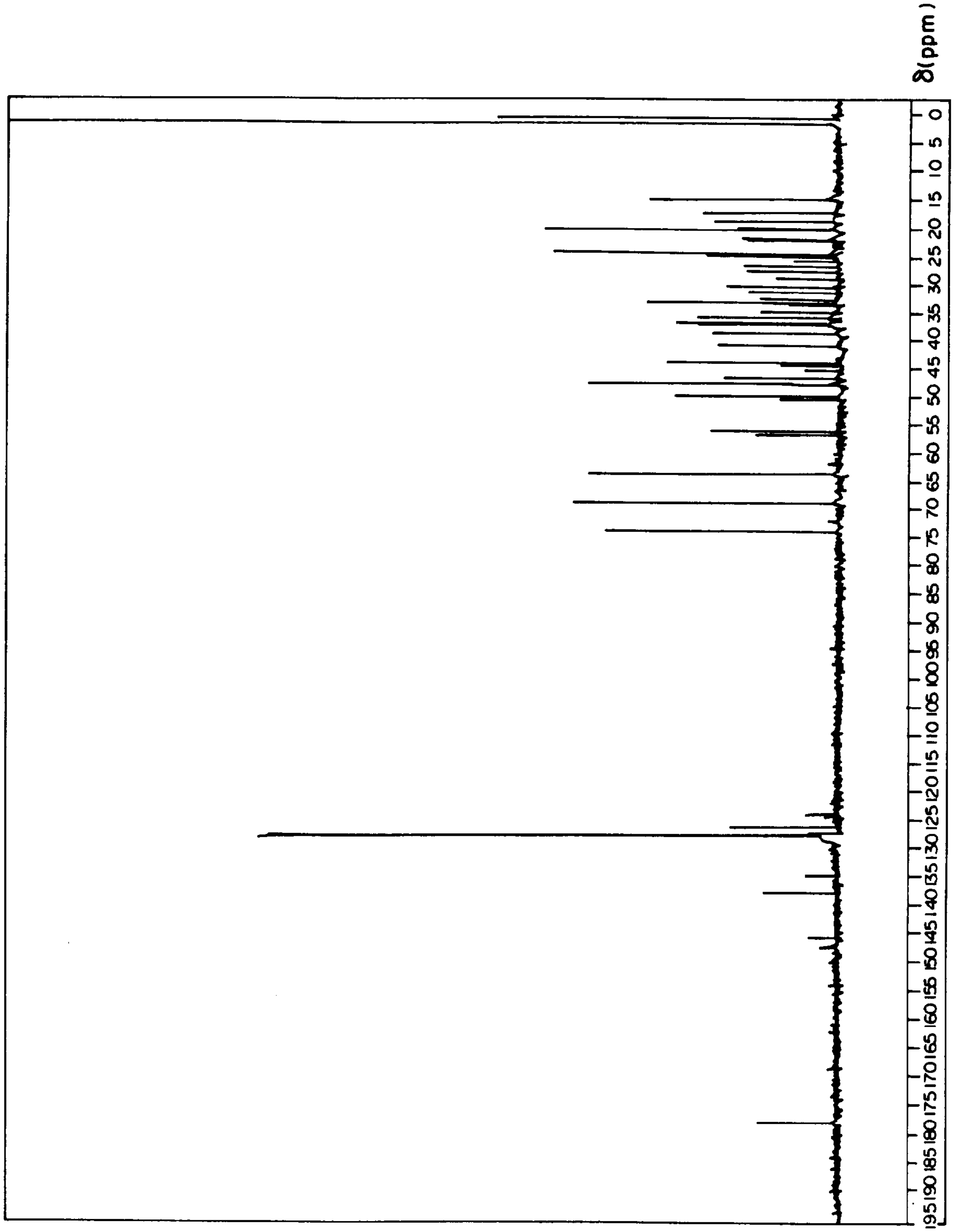
δ(ppm)
0 5 10 15 20 25 30 35 40 45 50 55 60 65 70 75 80 85 90 95 100 105 110 115 120 125 130 135 140 145 150 155 160 165 170 175 180 185 190 195

FIG. 7

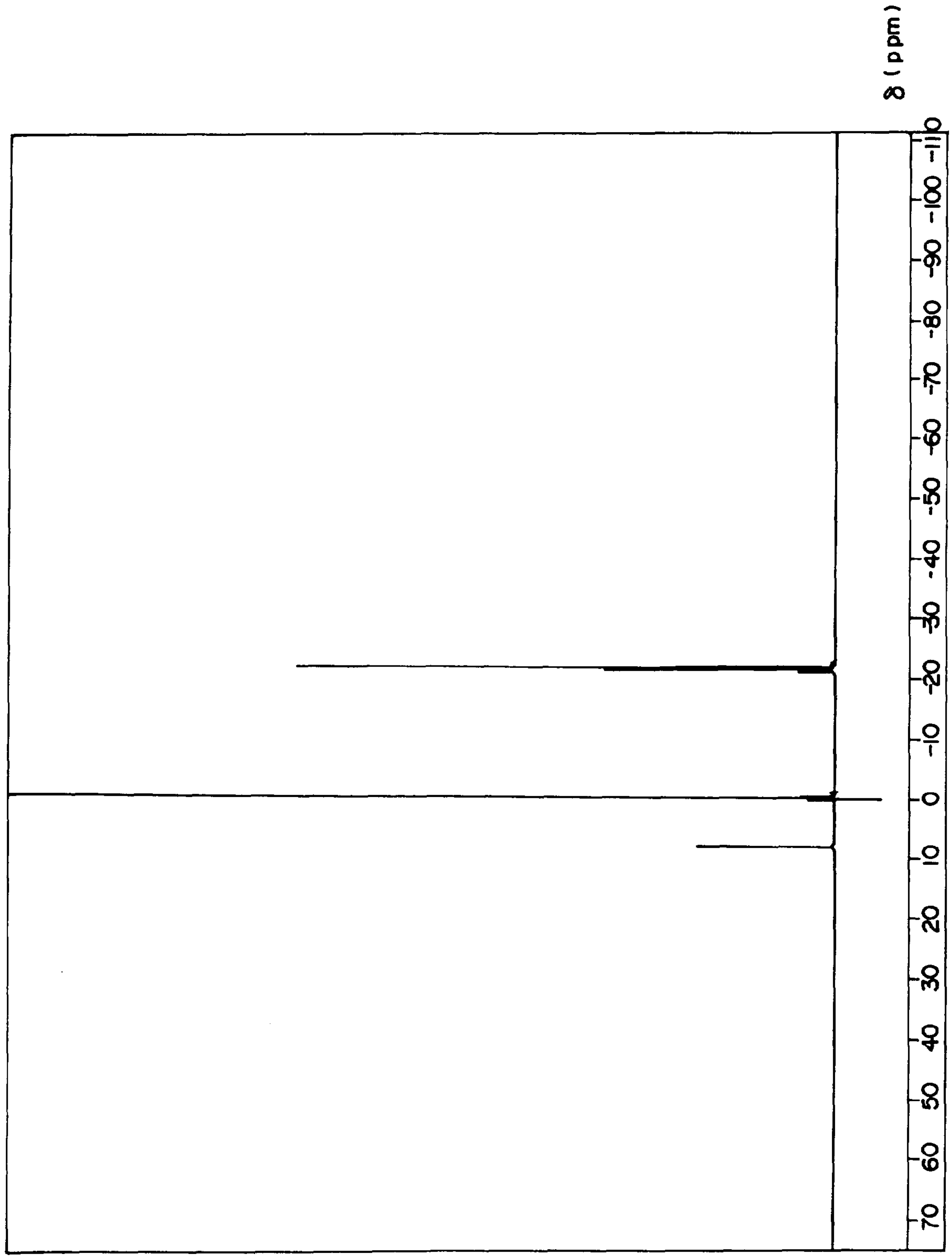


FIG.8

FIG. 9

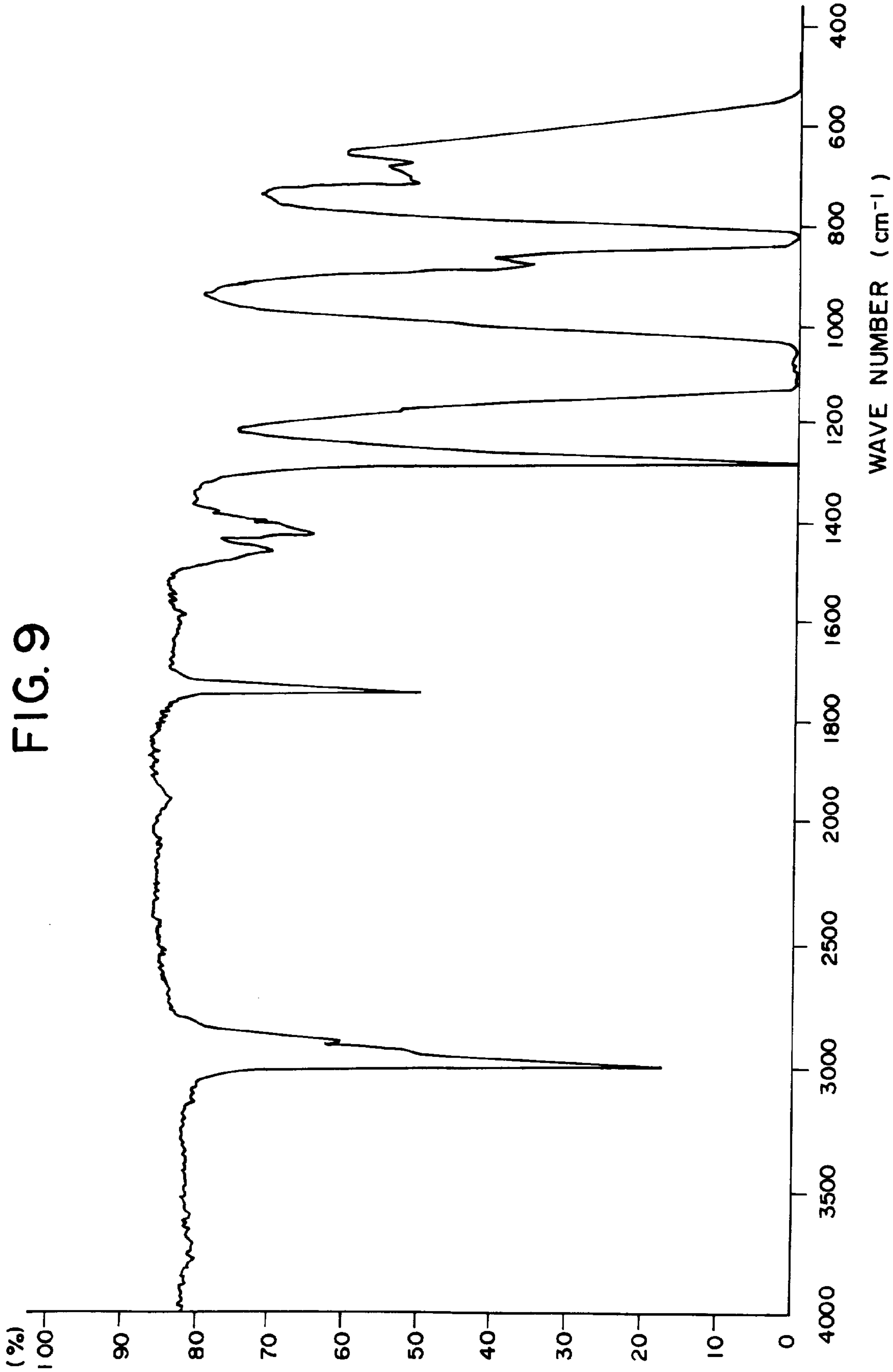
(%)
100
90
80
70
60
50
40
30
20
10
0
4000
3500
3000
2500
2000
1800
1600
1400
1200
1000
800
600
400
WAVE NUMBER (cm⁻¹)

FIG. 10

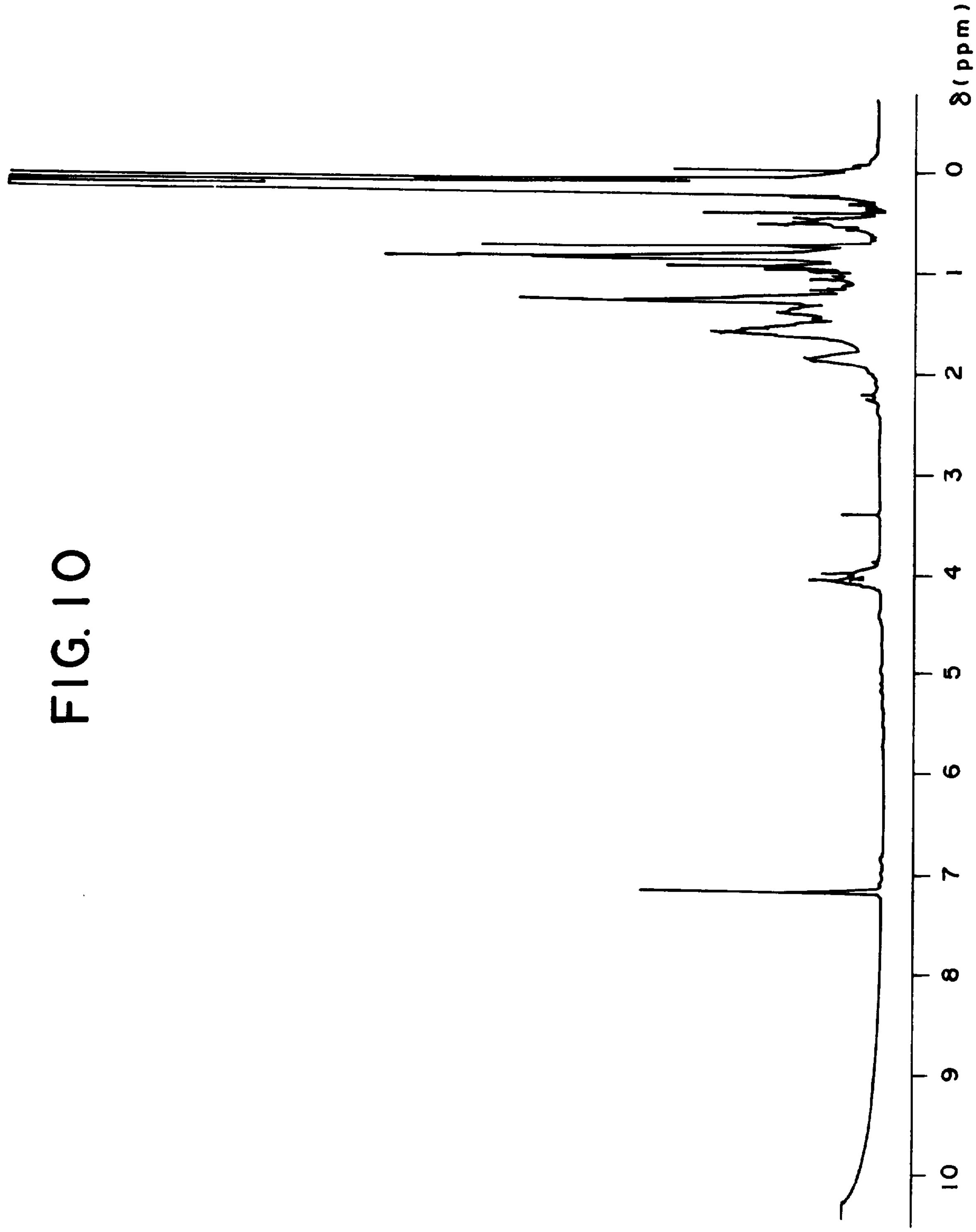

FIG. 11

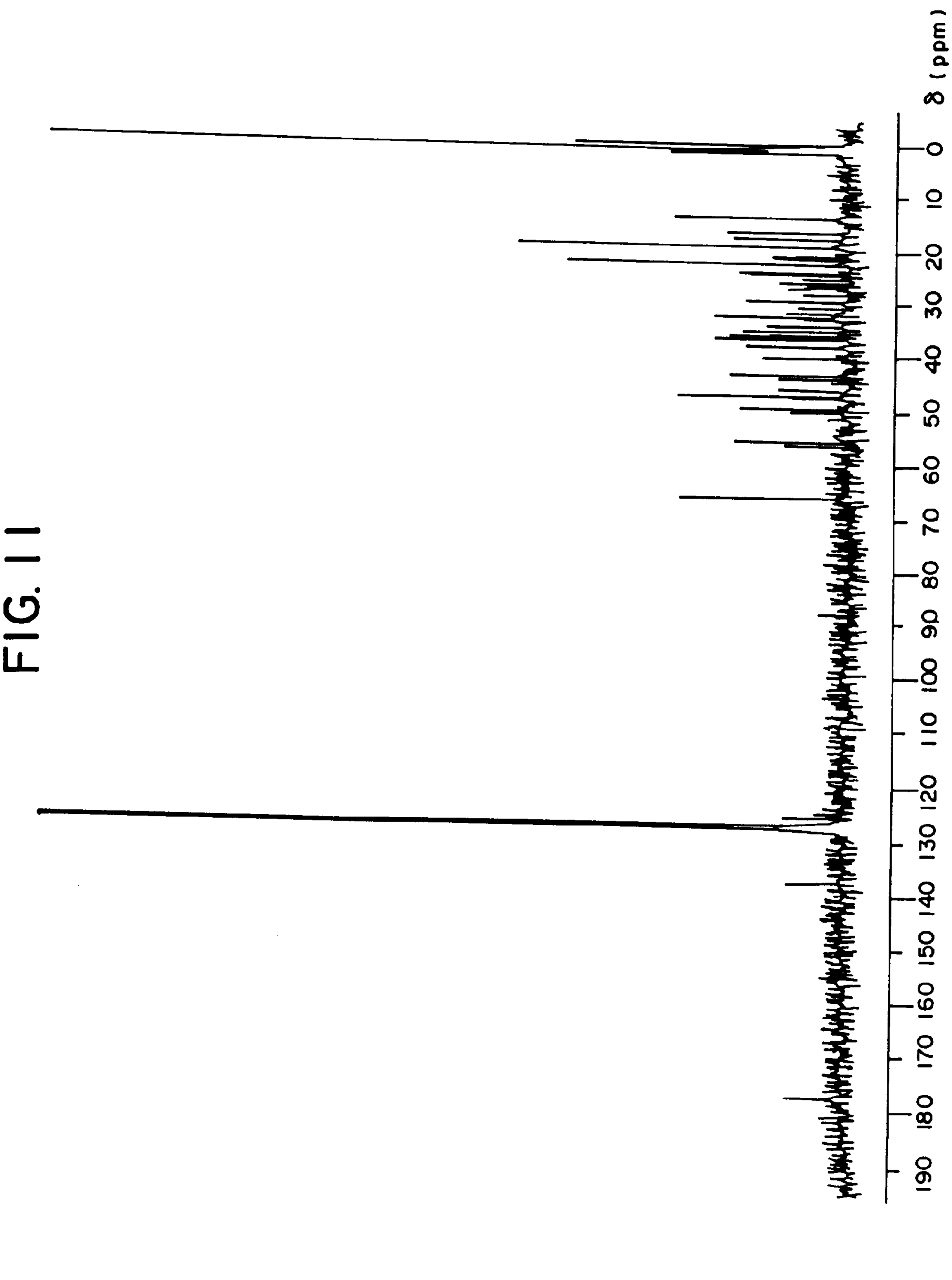
δ (ppm)
0
10
20
30
40
50
60
70
80
90
100
110
120
130
140
150
160
170
180
190

FIG. 12